# EUROPÄISCHE PATENTANMELDUNG

(11) **EP 0 962 222 A2**
(43) Veröffentlichungstag der Anmeldung: **08.12.1999**
(21) Anmeldenummer: 99110683.2
(22) Anmeldetag: 02.06.1999
(51) Int. Cl.: A61K 7/42

(54) **Verwendung von cholesterisch-flüssigkristallinen Zusammensetzungen als UV-Filter in kosmetischen und pharmazeutischen Zubereitungen**

(30) Priorität: 04.06.1998 DE 19824972
(71) Anmelder: BASF AKTIENGESELLSCHAFT, 67056 Ludwigshafen (DE)
(72) Erfinder: Schumacher, Peter Dr., 68163 Mannheim (DE); Schneider, Norbert, 67122 Altrip (DE); Westenfelder, Horst, 67435 Neustadt (DE); Haremza, Sylke Dr., 69151 Neckargemünd (DE); Habeck, Thorsten Dr., 67149 Meckenheim (DE); Meyer, Frank Dr., 69115 Heidelberg (DE)

(57) **Zusammenfassung**

Verwendung von cholesterisch-flüssigkristallinen Zusammensetzungen, enthaltend
a) mindestens ein chirales flüssigkristallines, polymerisierbares Monomeres der allgemeinen Formel I,

   [Z¹-Y¹-(A¹)ₘ-Y²-M¹-Y³-]ₙ-X I

   mit dem eine cholesterisch-flüissigkristalline Phase mit einer Ganghöhe von kleiner 450 nm erzielt werden kann,
   oder
b) eine Mischung aus
   b₁) mindestens einem achiralen flüssigkristallinen polymerisierbaren Monomeren der allgemeinen Formel II

      Z²-Y⁴-(A²)ₒ-Y⁵-M²-Y⁶-(A³)ₚ-Y⁷-Z³ II

      und
   b₂₎ mindestens einem chiralen Zusatzstoff, mit dem eine cholesterisch-flüssigkristalline Phase mit einer Ganghöhe von kleiner 450 nm erzielt werden kann,
in der die Variablen die in der Beschreibung erläuterte Bedeutung haben, als UV-Filter in kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

## Beschreibung

Die Erfindung betrifft die Verwendung von cholesterisch-flüssigkristallinen Zusammensetzungen als photostabile UV-Filter in kosmetischen und pharmazeutischen Zubereitungen zum Schutz der menschlichen Epidermis oder menschliche Haare gegen UV-Strahlung, speziell im Bereich von 280 bis 450 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstotfe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Dermatologische Untersuchungen haben gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A- und UV-B-Bereich notwendig erscheinen.

Neben den bekannten UV-Absorbern, wie z.B. 4-Methoxy-zimtsäure-2-ethylhexylester oder 3-(4 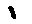 -Methyl)-benzyliden-bornan-2-on werden in kosmetischen und pharmazeutischen Formulierugen häufig auch Lichtschutzmittel eingesetzt, die in Form von Pigmenten die UV-Strahlen reflektieren bzw. absorbieren. Die wichtigsten dieser verwendeten Pigmente sind Titandioxid und Zinkoxid. Bei hohen Einsatzkonzentrationen kann mit Pigmenten eine vollständige Abdeckung der Haut erreicht werden. Dann reflektieren die Partikel allerdings nicht nur UV-Strahlung sondern auch sichtbares Licht, was die häufig nicht gewünschte starke Eigenfärbung pigment-haltiger Präparate bewirkt.

Während grobteilige Titandioxid Pigmente (Teilchengröße > 500 nm) im UV-B- und UV-A-Bereich vergleichbar wirken, verschiebt sich das Wirkungsspektrum bei feinteiligem Material mit abnehmender Teilchengröße in Richtung UV-B. Dies zeigt, daß die Absorptions-/Reflektionscharakteristik direkt von der Größe und der Verteilung der Teilchen abhängt. Für einen ausgewogenen UV-B- und UV-A-Schutz sind daher bestimmte Teilchengrößenverteilungen erforderlich.

Von Nachteil bei der Verwendung der oben genannten Pigmente erweist sich, daß während der Lagerung der kosmetischen oder pharmazeutischen Lichtschutzmittelformulierungen oftmals Agglomeration, Aggregation und/oder Separation der Pigmentteilchen stattfinden. Die Folge der hierdurch veränderten optischen Eigenschaften kann eine drastisch verringerte Lichtschutzwirkung sein.

Als Alternative zu den o.g. Pigmenten beschreibt DE-A-196 19 460 die Verwendung von Flüssigkristallmischungen mit cholesterischer Phase enthaltend a) flüssigkristalline Organosiloxane, die Dian-hydrohexit-Derivate als chirale Gruppen aufweisen und b) chirale monomere Zusatzstoffe, die die gleiche Helizität induzieren wie die jeweiligen flüssigkristallinen Organosiloxane, zur Herstellung von, für kosmetische Zwecke geeignete UV-Schutzschichten in Form von Filmen oder Plättchen. Die hier beschriebenen Flüssigkristallmischungen haben den Nachteil, daß sie sich aufgrund ihrer hohen Viskosität nur unbefriedigend zu Pigmenten verarbeiten lassen.

DE-A-196 29 761 beschreibt kosmetische oder pharmazeutische Zubereitungen, enthaltend Pigmente aus Polyorganosiloxanen mit vom Betrachtungswinkel abhängiger Farbigkeit. Bei den Pigmenten handelt es sich dabei um mindestens eine orientierte vernetzte Substanz einer flüssigkristallinen Struktur mit chiraler Phase. Die hier in den kosmetischen und pharmazeutischen Rezepturen offenbarten Pigmente besitzen zwar gewisse Absorptionseigenschaften im UV-Bereich. Sie haben jedoch für bestimmte Anwendungen den Nachteil, daß es sich hierbei um farbige Verbindungen handelt deren Einsatzgebiet dadurch eingeschränkt ist. Sehr häufig sind aber gerade solche kosmetischen und pharmazeutischen Zubereitungen gefragt, mit denen ein UV-Schutz erzielt wird, bei denen aber eine Färbung der Zubereitung unerwünscht ist.

Es bestand nun die Aufgabe, neue Lichtschutzmittel für kosmetische und pharmazeutische Zwecke vorzuschlagen, die im UV-A- und/oder UV-B-Bereich als Filter wirken und die in Form von Pigmenten die oben genannten Nachteile nicht aufweisen.

Diese Aufgabe wurde erfindungsgemäß gelöst durch Verwendung von cholesterisch-flüssigkristallinen Zusammensetzungen, enthaltend
a) mindestens ein chirales flüssigkristallines polymerisierbares Monomeres der allgemeinen Formel I,

   [Z¹-Y¹-(A¹)ₘ-Y²-M¹-Y³-]ₙ-X I

   mit dem eine cholesterisch-flüssigkristalline Phase mit einer Ganghöhe von kleiner 450 nm erzielt werden kann, in der die Variablen unabhängig voneinander folgende Bedeutung haben:
   - A¹: ein Spacer einer Kettenlänge von 1 bis 30 C-Atomen,
   - Y¹ bis Y³: eine chemische Bindung, -O-, -S-, -C(=O)-O-, -O-C(=O)-, -CH=CH-C(=O)-O-, -O-C(=O)-O-, -C(=O)-N(R)- oder -(R)N-C(=O)-, -CH₂-O-, -O-CH₂-, -CH=N-, -N=CH- oder -N=N-
   - M¹: eine mesogene Gruppe,
   - R: Wasserstoff, C₁-C₄-Alkyl,
   - Z¹: Wasserstoff, C₁-C₄-Alkyl, eine polymerisierbare Gruppe oder ein Rest, der eine polymerisierbare Gruppe trägt,
   - X: ein n-wertiger chiraler Rest,
   - m: 0 oder 1,
   - n: 1 bis 6
   wobei die Reste Z¹, Y¹, Y², Y³, A¹ und M¹ gleich oder verschieden sein können und mindestens ein Rest Z¹ eine polymerisierbare Gruppe oder einen Rest, der eine polymerisierbare Gruppe enthält, darstellt, wenn n größer als 1 ist,
   oder
b) eine Mischung aus
   b₁) mindestens einem achiralen flüssigkristallinen polymerisierbaren Monomeren der allgemeinen Formel II

      Z²-Y⁴-(A²)ₒ-Y⁵-M²-Y⁶-(A³)p-Y⁷-Z³ II

      in der die variablen unabhängig voneinander folgende Bedeutung haben:
      - A² und A³: ein Spacer einer Kettenlänge von 1 bis 30 C-Atomen,
      - M²: eine mesogene Gruppe,
      - Y⁴ bis Y⁷: eine chemische Bindung, -O-, -S-, -C(=O)-O-, -O-C(=O)-, -CH=CH-C(=O)-O-, -O-C(=O)-O-, -C(=O)-N(R¹)- oder -(R¹)N-C(=O)-, -CH₂-O-, -O-CH₂-, -CH=N-, -N=CH- oder -N=N-,
      - R¹: Wasserstoff, C₁-C₄-Alkyl,
      - o,p: 0 oder 1,
      - Z² und Z³: Wasserstoff, C₁-C₄-Alkyl, eine polymerisierbare Gruppe oder ein Rest, der eine polymerisierbare Gruppe trägt,
      wobei mindestens eine der variablen Z² oder Z³ eine polymerisierbare Gruppe oder einen Rest, der eine polymerisierbare Gruppe trägt, darstellt,
      und
   b₂) mindestens einen chiralen Zusatzstoff, mit dem eine cholesterisch-flüssigkristalline Phase mit einer Ganghöhe von kleiner 450 nm erzielt werden kann,
als UV-Filter in kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

Als Spacer A¹ kommen alle für diesen Zweck bekannten Gruppen in Betracht. Die Spacer enthalten in der Regel 1 bis 30, vorzugsweise 1 bis 12 , besonders bevorzugt 1 bis 6 C-Atome und bestehen aus vorwiegend linearen aliphatischen Gruppen. Sie können in der Kette, z.B. durch nicht benachbarte Sauerstoff- oder Schwefelatome oder Imino- oder Alkyliminogruppen wie beispielsweise Methyliminogruppen, unterbrochen sein. Als Substituenten für die Spacerkette kommen dabei noch Fluor, Chlor, Brom, Cyan, Methyl und Ethyl in Betracht.

Repräsentative Spacer sind beispielsweise:
-(CH₂)_{q}-, -(CH₂CH₂O)ᵣCH₂CH₂-, -CH₂CH₂SCH₂CH₂-, -CH₂CH₂NHCH₂CH₂-, 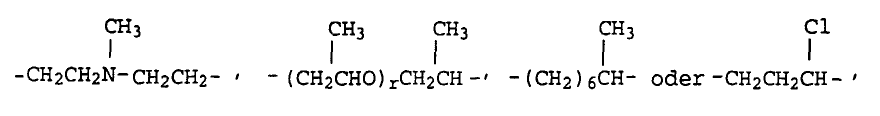 wobei r für 1 bis 3 und g für 1 bis 12 steht.

Bevorzugte Spacer sind Ethylen, Propylen, n-Butylen, n-Pentylen und n-Hexylen.

Es ist aber auch möglich, die mesogene Gruppe direkt mit dem Rest Z¹ zu verknüpfen. In diesen Fall stehen m für 0 sowie Y¹ und Y² gemeinsam für eine chemische Bindung.

Als Reste M¹ können alle bekannten mesogenen Gruppen dienen.

Insbesondere kommen mesogene Gruppen der Formel

(-T-Y⁸)ₛ-T-

in Betracht, in der die Variablen folgende Bedeutung haben:
- T: gleiche oder verschiedene zweiwertige gesättigte oder ungesättigte iso- oder heterocyclische Reste,
- Y⁸: Gruppen der Definition für Y¹ bis Y⁷, s 0, 1, 2 oder 3,
wobei im Falle s > O, sowohl die Reste T als auch die Gruppen Y⁸ jeweils untereinander gleich oder verschieden sein können.

Vorzugsweise ist s gleich 1 oder 2.

Die Reste T können auch durch Fluor, Chlor, Brom, Cyan, Hydroxy oder Nitro substituierte Ringsysteme sein. Bevorzugte Reste T sind:

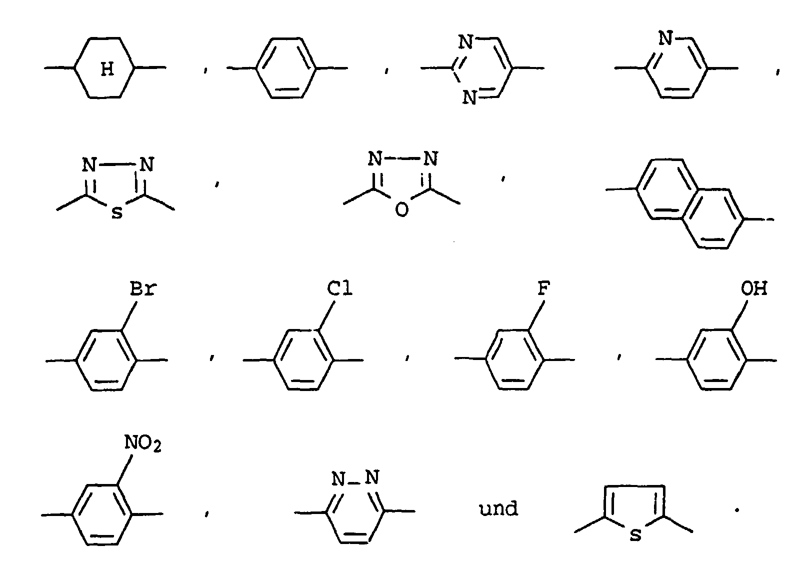

Bevorzugt als mesogene Gruppen M¹ sind z.B.:

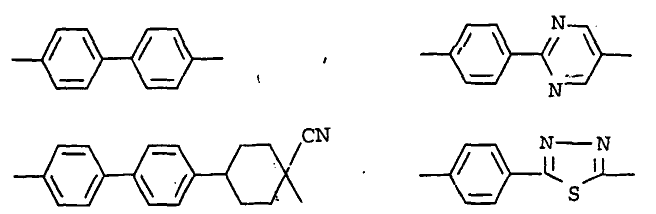

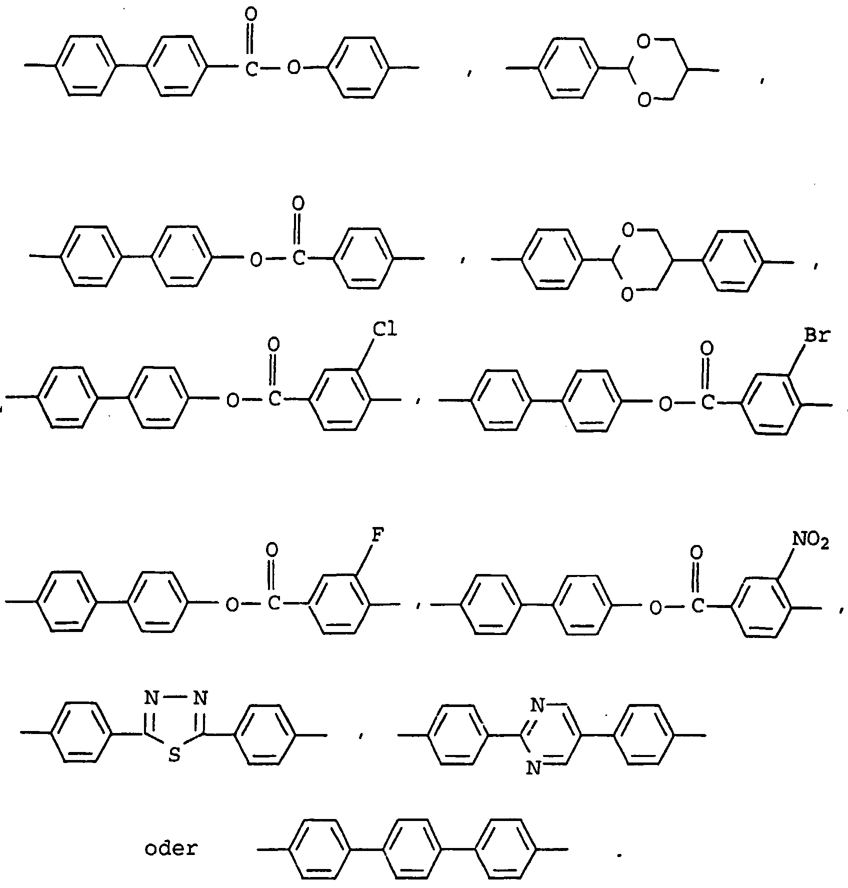

Besonders bevorzugt sind mesogene Gruppen M¹ der folgenden Formeln 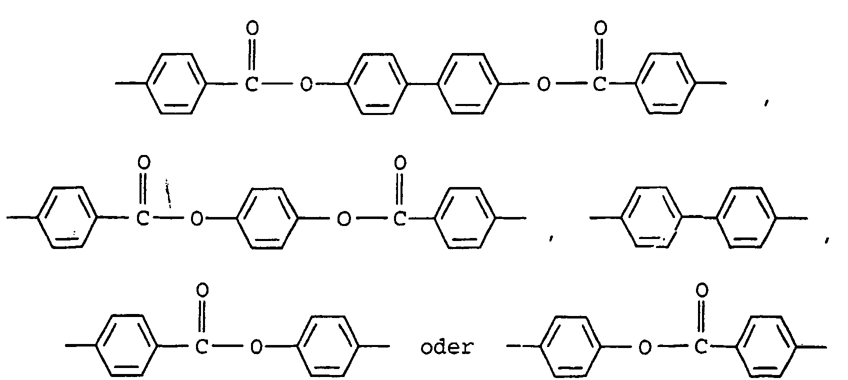 wobei jeder aromatische Ring bis zu drei gleiche oder verschiedene Substituenten aus der folgenden Gruppe tragen kann:

Wasserstoff, C₁-C₂₀-Alkyl, C₁-C₂₀-Alkoxy, C₁-C₂₀-Alkoxycarbonyl, C₁-C₂₀-Monoalkylaminocarbonyl, C₁-C₂₀--Alkylcarbonyl, C₁-C₂₀-Alkylcarbonyloxy, C₁-C₂₀-Alkylcarbonylamino, Formyl, Halogen, Cyan, Hydroxy oder Nitro.

Bevorzugte Substituenten für die aromatischen Ringe sind neben Fluor, Chlor, Brom, Cyan, Formyl und Hydroxy vor allem kurzkettige aliphatische Reste wie Methyl, Ethyl, n-Propyl, i-Propyl, n-Butyl, i-Butyl, sek.-Butyl, tert.-Butyl sowie Alkoxy-, Alkoxycarbonyl-, Alkylcarbonyl-, Alkylcarbonyloxy-, Alkylcarbonylamino- und Monoalkylaminocarbonylreste, die diese Alkylgruppen enthalten.

Die äußeren Benzolringe der besonders bevorzugten Gruppen M¹ haben vorzugsweise folgende Substitutionsmuster: 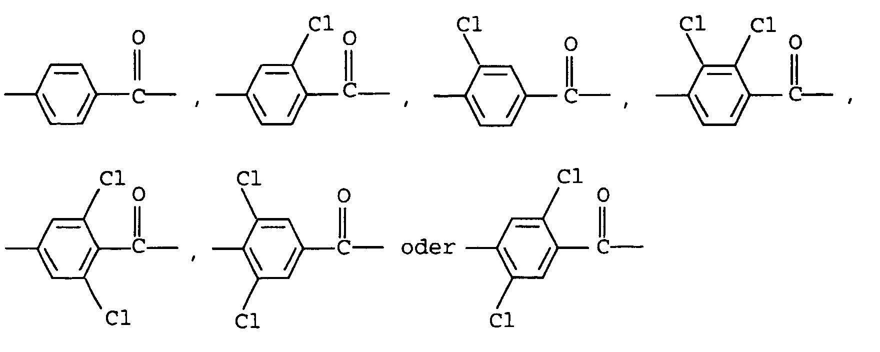 oder sie sind analog mit F, Br, CH₃, OCH₃, CHO, COCH₃, OCOCH₃ oder CN anstelle von C1 substituiert, wobei die Substituenten auch gemischt vorliegen können. Ferner sind die Strukturen 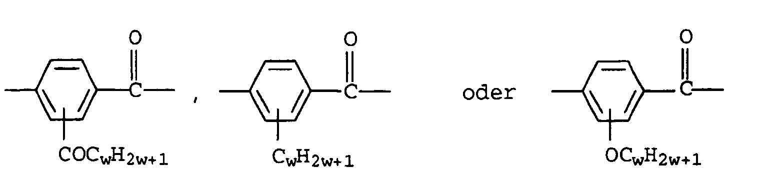 zu nennen, bei denen w 2 bis 20, vorzugsweise 8 bis 15, bedeutet.

Die bevorzugten Substitutionsmuster des mittleren Benzolrings der besonders bevorzugten Gruppen M¹ sind

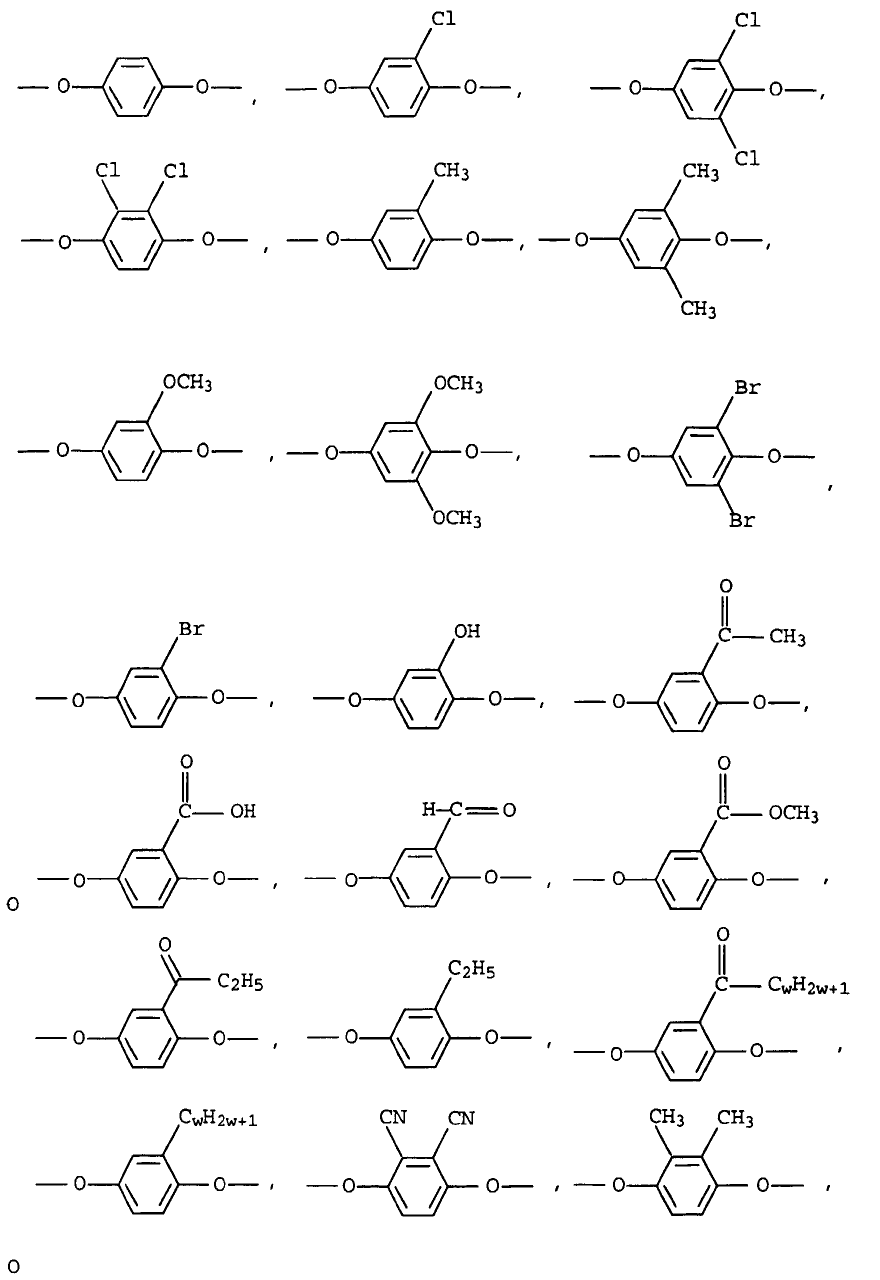

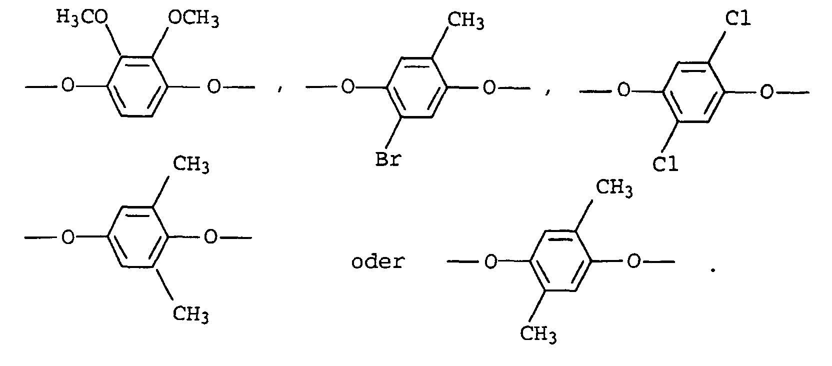

Bevorzugte Reste für Z¹ sind: 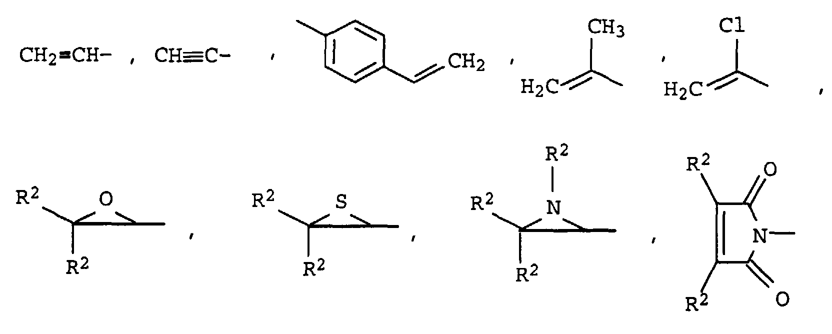 -N=C=O, -N=C=S, -O-C≡N, -COOH, -OH oder NH₂ wobei die Reste R² gleich oder verschieden sein können und Wasserstoff oder C₁-C₄-Alkyl, wie Methyl, Ethyl, n-Propyl, iso-Propyl, n-Butyl, iso-Butyl, sec.-Butyl oder tert.-Butyl bedeuten. Von den reaktiven polymerisierbaren Gruppen können die Cyanate spontan zu Cyanuraten trimerisieren und sind daher bevorzugt. Die anderen genannten Gruppen benötigen zur Polymerisation weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Carboxylgruppen können zu Polyestern und Polyamiden kondensiert werden. Die Maleinimidogruppe eignet sich besonders zur radikalischen Copolymerisation mit olefinischen Verbindungen wie Styrol. Die komplementären reaktiven Gruppen können dabei entweder in einer zweiten erfindungsgemäßen Verbindung vorhanden sein, die mit der ersteren gemischt wird, oder sie können durch Hilfsverbindungen, die 2 oder mehr dieser komplementären Gruppen enthalten, in das polymere Netzwerk eingebaut werden.

Besonders bevorzugte Gruppierungen Z¹-Y¹ sind Acrylat und Methacrylat.

Y¹-Y³ können die oben genannten Bedeutungen haben, wobei unter einer chemischen Bindung eine kovalente Einfachbindung verstanden werden soll.

Als Alkylreste seien für R und Z¹ verzweigte oder unverzweigte C₁-C₄-Alkylketten, bevorzugt Methyl, Ethyl, n-Propyl, 1-Methyl-ethyl, n-Butyl, 1-Methylpropyl-, 2-Methylpropyl oder 1,1-Dimethylethyl genannt.

Von den chiralen Resten X der Verbindungen der Formel I sind u.a. aufgrund der Verfügbarkeit insbesondere solche bevorzugt, die sich von Zuckern, Binaphthyl- oder Biphenylderivaten sowie optisch aktiven Glykolen, Dialkoholen oder Aminosäuren ableiten. Bei den Zuckern sind insbesondere Pentosen und Hexosen und davon abgeleitete Derivate zu nennen.

Beispiele für Reste X sind die folgenden Strukturen, wobei die endständigen Striche jeweils die freien Valenzen bedeuten.

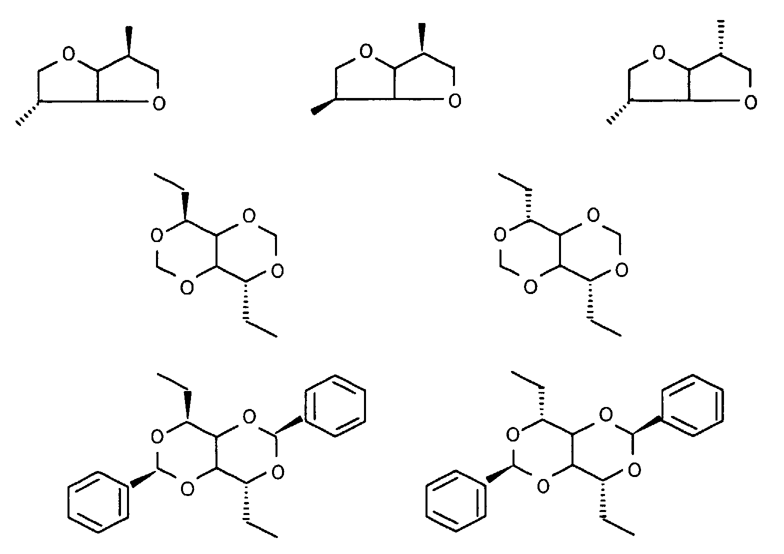

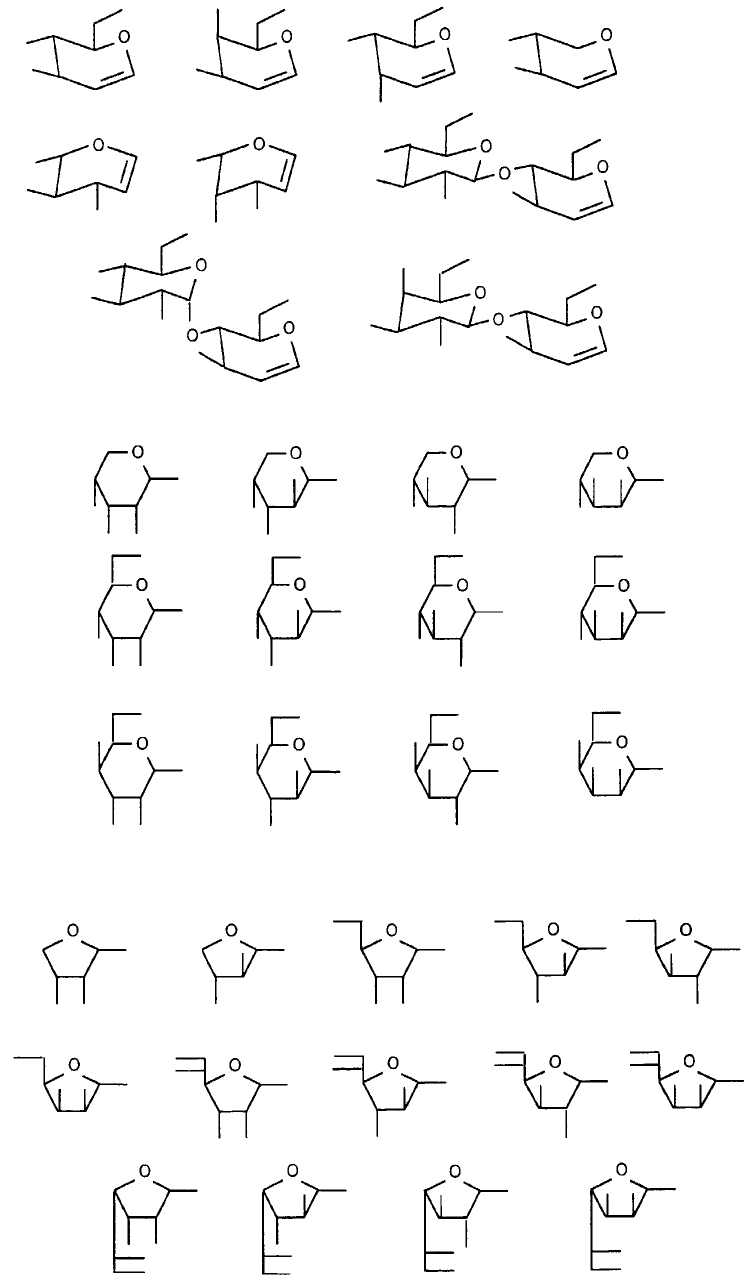

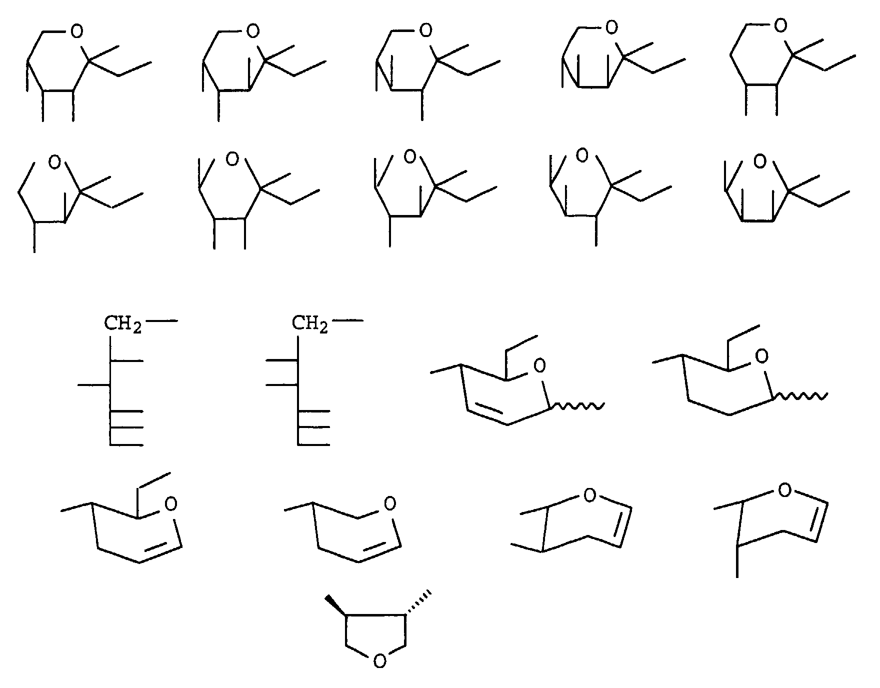

Besonders bevorzugt sind

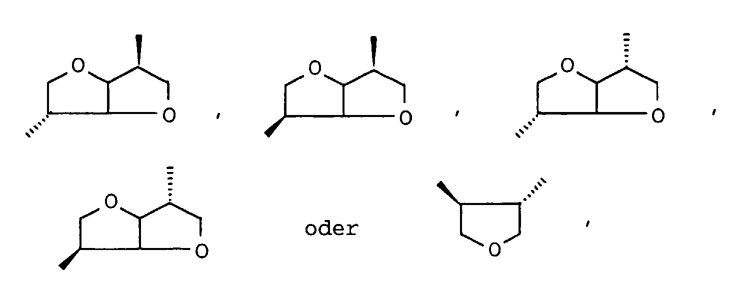

Weiterhin sind auch chirale Gruppen geeignet, die folgende Strukturen aufweisen:

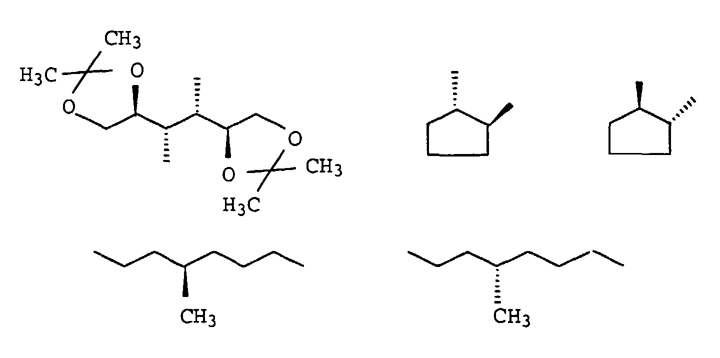

Weitere Beispiele sind in der deutschen Anmeldung P 43 42 280.2 aufgeführt.

m ist bevorzugt 1 und n steht bevorzugt für 2.

Als Komponente b₁) enthält das polymerisierbare Gemisch b) für die erfindungsgemäße Verwendung mindestens ein achirales flüssigkristallines polymerisierbares Monomer der Formel II

Z²-Y⁴-(A²)ₒ-Y⁵-M²-Y⁶-(A³)ₚ-Y⁷-Z³ II

in der die variablen unabhängig voneinander die folgende Bedeutung haben:
- A² und A³: ein Spacer einer Kettenlänge von 1 bis 30 C-Atomen,
- M²: eine mesogene Gruppe,
- Y⁴ bis: Y⁷
eine chemische Bindung, -O-, -S-, -C(=O)-O-, -O-C(=O)-, -CH=CH-C(=O)-O-, -O-C(=O)-O-, -C(=O)-N(R¹)- oder -(R¹)N-C(=O)-, -CH₂-O-, -O-CH₂-, -CH=N-, -N=CH- oder -N=N-,
- R¹: Wasserstoff, C₁-C₄-Alkyl,
- o,p: 0 oder 1
- Z² und Z³: Wasserstoff, C₁-C₄-Alkyl, eine polymerisierbare Gruppe oder ein Rest, der eine polymerisierbare Gruppe trägt,
wobei mindestens eine der variablen Z² oder Z³ eine polymerisierbare Gruppe oder einen Rest, der eine polymerisierbare Gruppe trägt, darstellt.

Dabei gelten für die polymerisierbaren Gruppen, die Brückenglieder Y⁴ bis Y⁷ die Spacer und die mesogene Gruppe die gleichen Bevorzugungen wie für die entsprechenden variablen der Formel I.

Ebenso wie in Formel I ist auch möglich, die mesogene Gruppe direkt mit den Resten Z² oder Z³ zu verknüpfen. In diesen Fällen stehen o und/oder p für 0 sowie Y⁴ und Y⁵ und/oder Y⁶ und Y⁷ gemeinsam für eine chemische Bindung.

Außerdem enthält das Gemisch b) noch einen chiralen Zusatzstoff b₂).

Als chirale Dotierstoffe für flüssigkristalline Phasen sind zahlreiche Verbindungen bekannt (z.B. aus DE-A 43 42 280 und DE-A 196 11 101). Geeignete Dotierstoffe sollten eine hohe Verdrillungsfähigkeit besitzen, so daß geringe Mengen des Dotierstoffs zur Induktion der helikalen Struktur ausreichen. Außerdem sollten die chiralen Dotierstoffe eine gute Kompatibilität zu den flüssigkristallinen Verbindungen zeigen, so daß eine effektive Wechselwirkung zwischen diesen Komponenten ermöglicht wird.

Das Ausmaß der Verdrillung hängt jeweils von der Verdrillungsfähigkeit des chiralen Dotierstoffs und von seiner Konzentration ab. Damit hängt also die Ganghöhe der Helix und wiederum auch die Interferenzwellenlänge von der Konzentration des chiralen Dotierstoffs ab. Es kann daher für den Dotierstoff kein allgemeingültiger Konzentrationsbereich angegeben werden. Der Dotierstoff wird in der Menge zugegeben, mit der die gewünschte UV-Reflektion erzielt wird.

Bevorzugte chirale Zusatzstoffe für b₂) sind Verbindungen der Formel III

[Z¹-Y¹-(A¹)ₘ-Y²-M³-Y³-]ₙ-X III

in der Z¹, Y¹, Y², Y³, A¹, X, m und n die obengenannten Bedeutung haben und M³ ein zweiwertiger Rest ist, der mindestens ein hetero- oder isocyclisches Ringsystem enthält.

Der Molekülteil M³ ähnelt dabei den beschriebenen mesogenen Gruppen, da auf diese Weise eine besonders gute Kompatibilität mit der flüssigkristallinen Verbindung erreicht wird. M³ muß jedoch nicht mesogen sein, da die Verbindung III lediglich durch ihre chirale Struktur eine entsprechende Verdrillung der flüssigkristallinen Phase bewirken soll. Bevorzugte Ringsysteme, die in M³ enthalten sind, sind die oben erwähnten Strukturen T, bevorzugte Strukturen M³ solche der oben genannten Formel (T-Y⁸)ₛ-T. Weitere Monomere und chirale Verbindungen der Gruppe b) sind in der WO 97/00600 und der ihr zugrundeliegenden DE-A-195 324 08 beschrieben, auf die hiermit in vollem Umfang Bezug genommen wird.

Die für die Verwendung als UV-Filter in kosmetischen und pharmazeutischen Zubereitungen bevorzugt verwendeten cholesterisch-flüssigkristallinen Zusammensetzungen enthalten eine Mischung aus mindestens einem achiralen flüssigkristallinen polymerisierbaren Monomeren der Formel II und mindestens einem chiralen polymerisierbaren Monomeren der Formel III.

Als besonders bevorzugte Monomere II sind folgende Strukturen zu nennen: 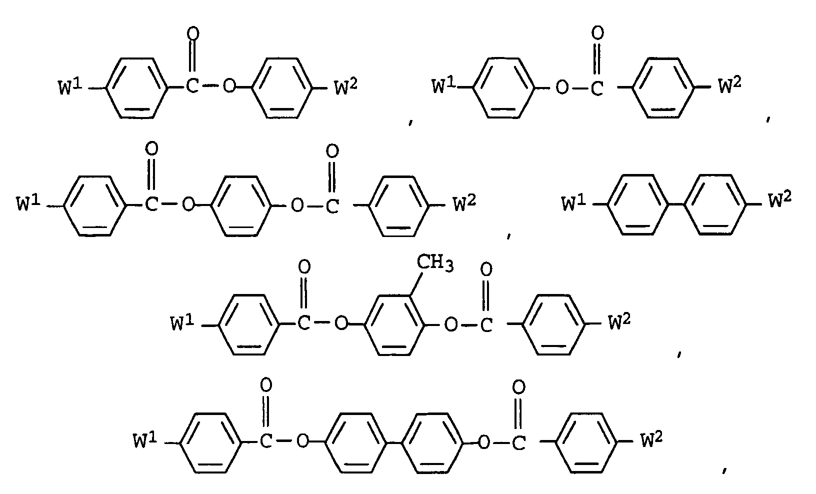
W¹: CH₂=CH-C(=O)-O-(CH₂)₄-O- , W²: -O-(CH₂)₄-O-C(=O)-CH=CH₂ 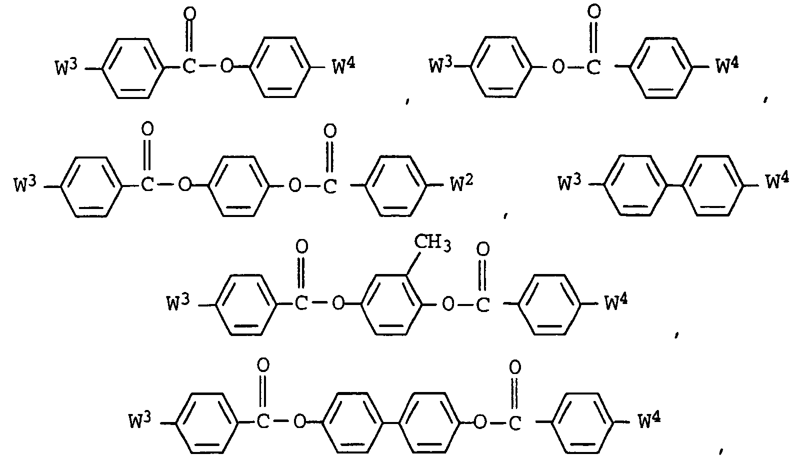
W³: CH₂=CH-C(=O)-O-(CH₂)₆-O- , W⁴: -O-(CH₂)₆-O-C(=O)-CH=CH₂ 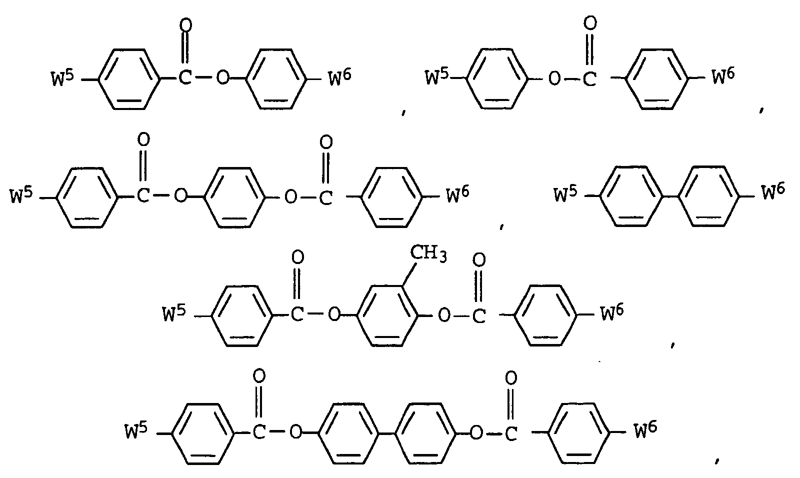
W⁵: CH₂=C(CH₃)-C(=O)-O-(CH₂)₄-O-, W⁶: -O-(CH₂)₄-O-C(=O)-C(CH₃)=CH₂ 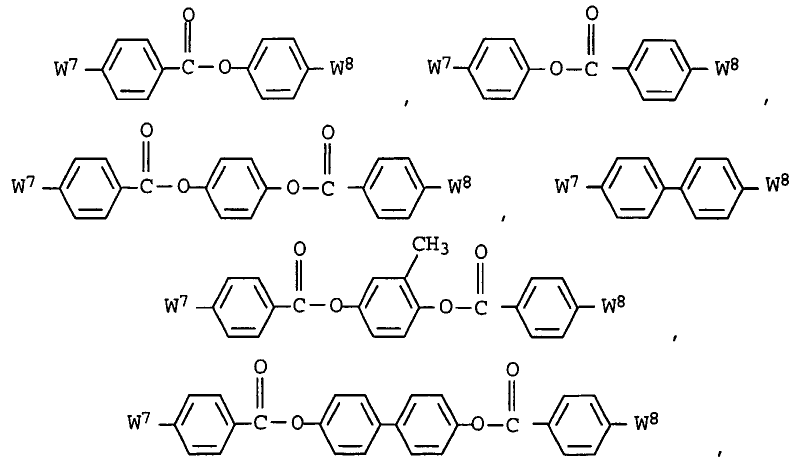
W⁷: CH₂=C(CH₃)-C(=O)-O-(CH₂)₆-O-, W⁸: -O-(CH₂)₆-O-C(=O)-C(CH₃)=CH₂ 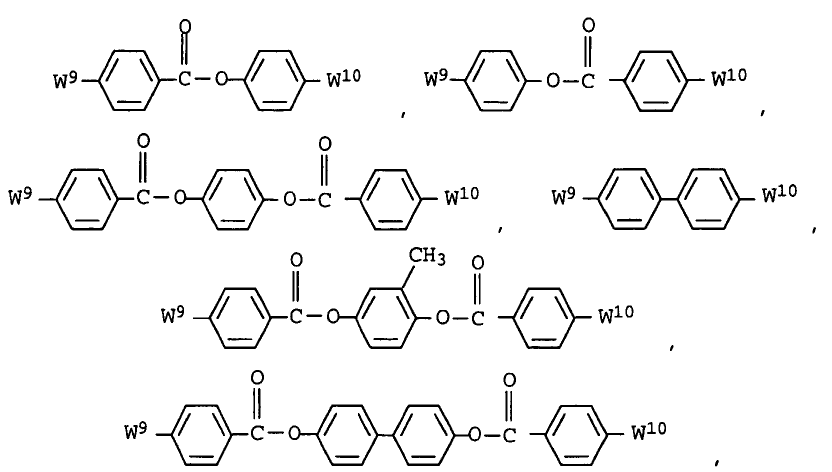
W⁹: CH₂=CH-C(=O)-O-(CH₂)₄-O-C(=O)-O- ,
W¹⁰: -O-(O=)C-O-(CH₂)₄-O-C(=O)-CH=CH₂ 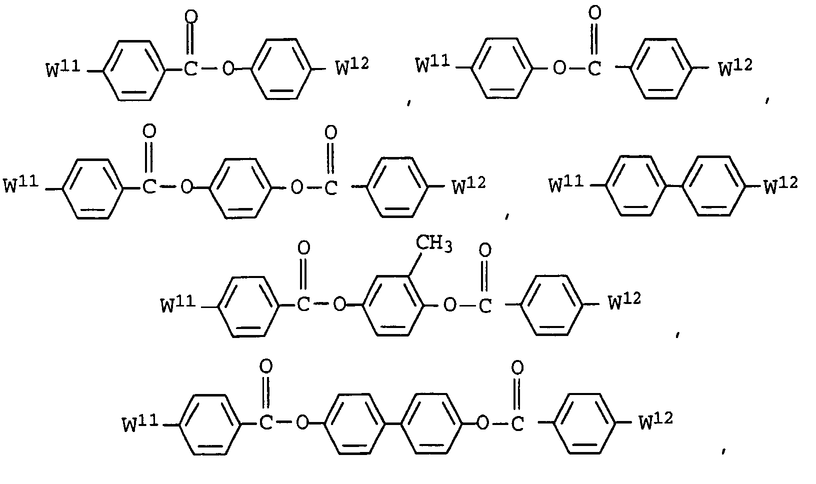
W¹¹: CH₂=CH-C(=O)-O-(CH₂)₆-O-C(=O)-O- ,
W¹²: -O-(O=)C-O-(CH₂)₆-O-C(=O)-CH=CH₂ 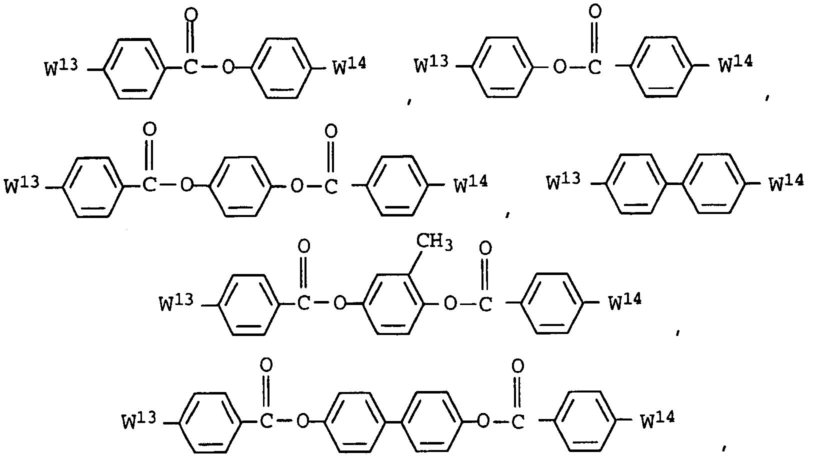
W¹³: CH₂=C(CH₃)-C(=O)-O-(CH₂)₄-O-C(=O)-O- ,
W¹⁴: -O-(O=)C-O-(CH₂)₄-O-C(=O)-C(CH₃)=CH₂ 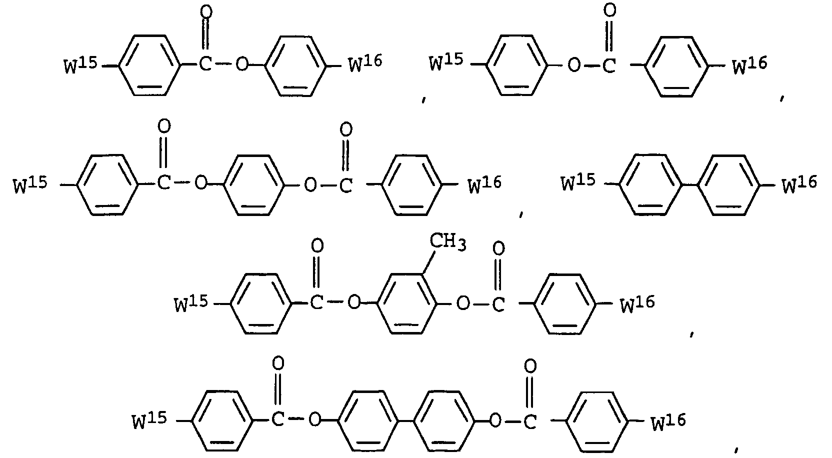
W¹⁵: CH₂=C(CH₃)-C(=O)-O-(CH₂)₆-O-C(=O)-O- ,
W¹⁶: -O-(O=)C-O-(CH₂)₆-O-C(=O)-C(CH₃)=CH₂

Als besonders bevorzugte Monomere III sind folgende Strukturen zu nennen: 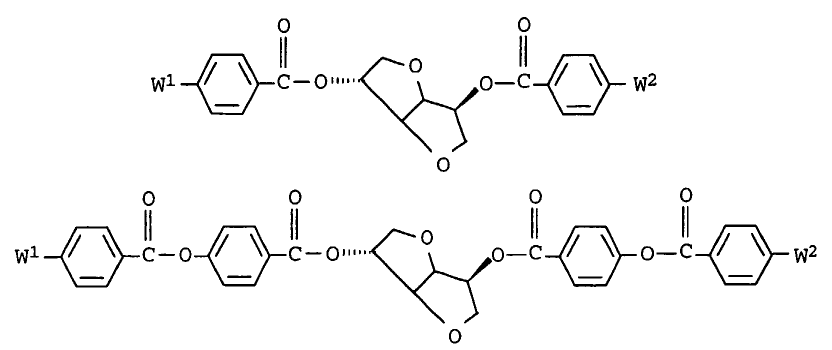
W¹: CH₂=CH-C(=O)-O-(CH₂)₄-O- W²: -O-(CH₂)₄-O-C(=O)-CH=CH₂ 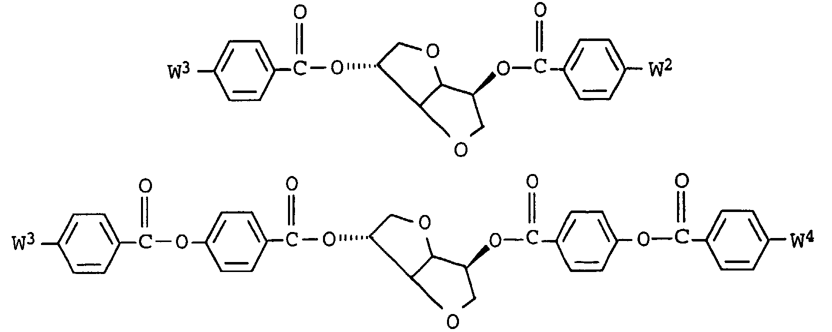
W³: CH₂=CH-C(=O)-O-(CH₂)₆-O- W⁴: -O-(CH₂)₆-O-C(=O)-CH=CH₂ 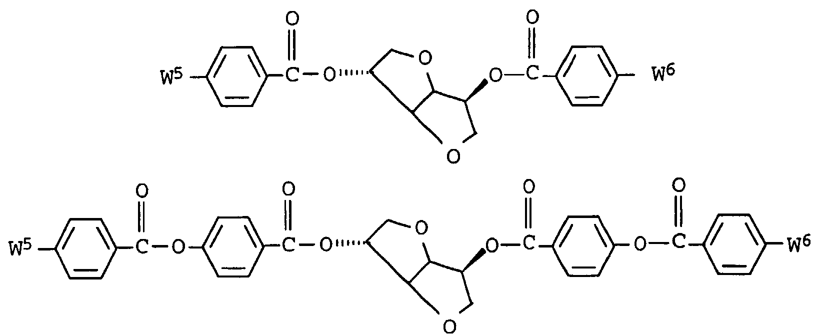
W⁵: CH₂=C(CH₃)-C(=O)-O-(CH₂)₄-O-, W⁶: -O-(CH₂)₄-O-C(=O)-C(CH₃)=CH₂ 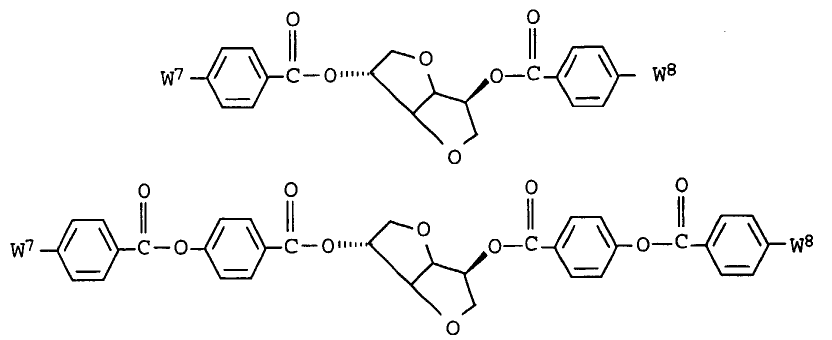
W⁷: CH₂=C(CH₃)-C(=O)-O-(CH₂)₆-O-, W⁸: -O-(CH₂)₆-O-C(=O)-C(CH₃)=CH₂ 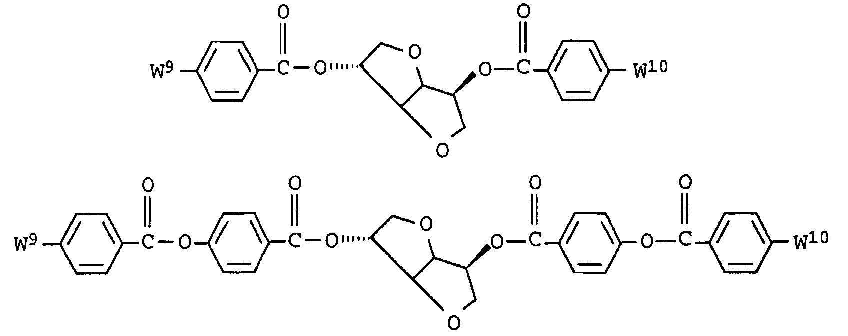
W⁹: CH₂=CH-C(=O)-O-(CH₂)₄-O-C(=O)-O-
W¹⁰: -O-(O=)C-O-(CH₂)₄-O-C(=O)-CH=CH₂ 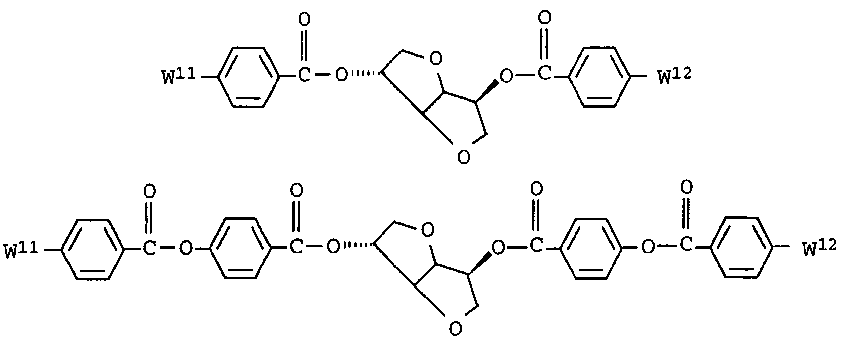
W¹¹: CH₂=CH-C(=O)-O-(CH₂)₆-O-C(=O)-O-
W¹²: -O-(O=)C-O(CH₂)₆-O-C(=O)-CH=CH₂ 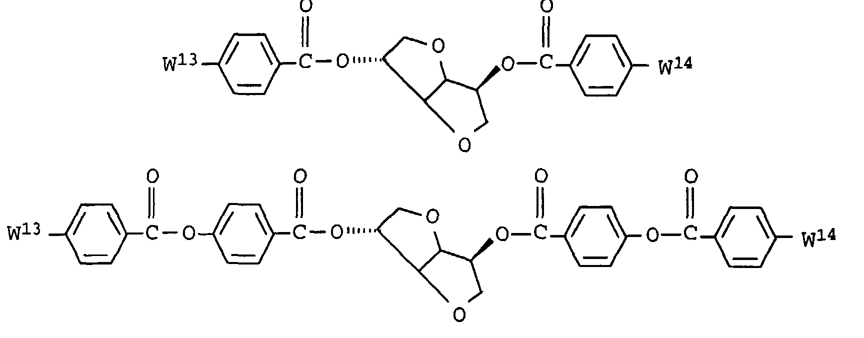
W¹³: CH₂=C(CH₃)-C(=O)-O-(CH₂)₄-O-C(=O)-O-,
W¹⁴: -O-(O=)C-O-(CH₂)₄-O-C(=O)-C(CH₃)=CH₂ 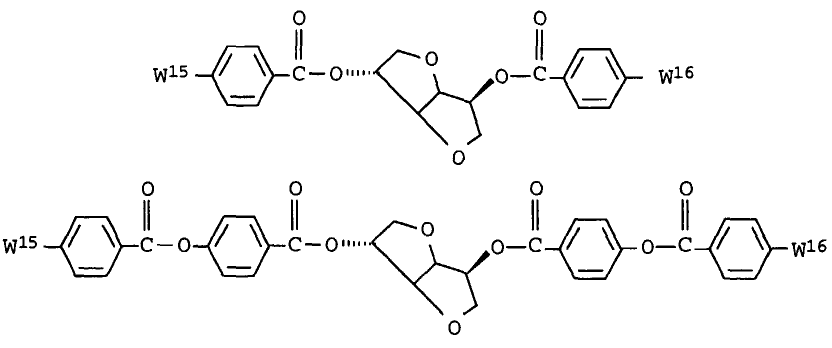
W¹⁵: CH₂=C(CH₃)-C(=O)-O-(CH₂)₆-O-C(=O)-O-,
W¹⁶: -O-(O=)C-O-(CH₂)₆-O-C(=O)-C(CH₃)=CH₂ 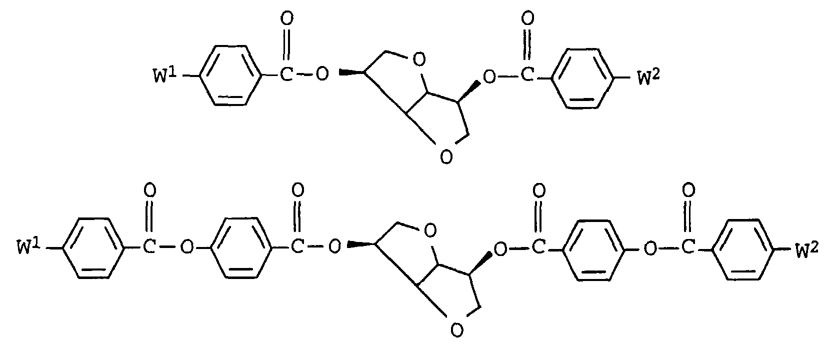
W¹: CH₂=CH-C(=O)-O-(CH₂)₄-O- W²: -O-(CH₂)₄-O-C(=O)-CH=CH₂ 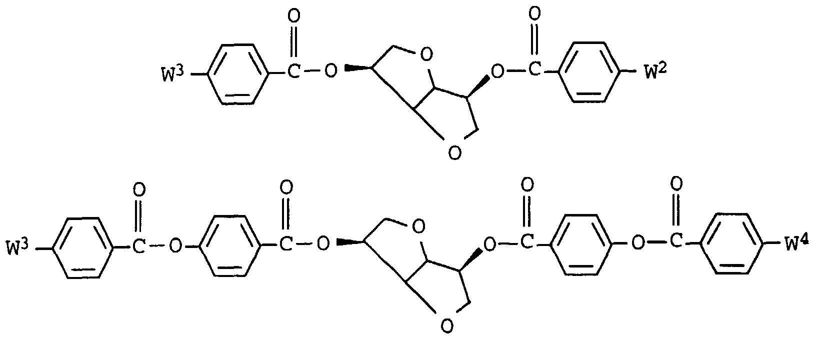
W³: CH₂=CH-C(=O)-O-(CH₂)₆-O- W⁴: -O-(CH₂)₆-O-C(=O)-CH=CH₂ 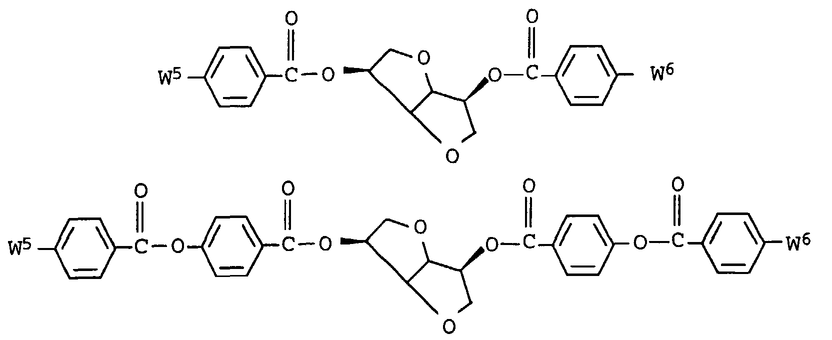
W⁵: CH₂=C(CH₃)-C(=O)-O-(CH₂)₄-O-, W⁶: -O-(CH₂)₄-O-C(=O)-C(CH₃)=CH₂ 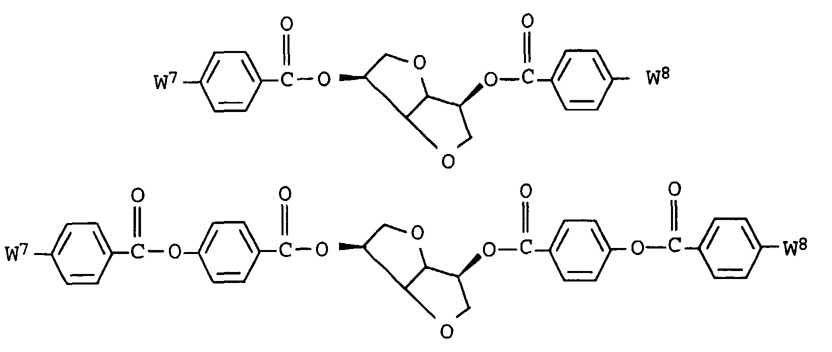
W⁷: CH₂=C(CH₃)-C(O)-O-(CH₂)₆-O-, W⁸: -O-(CH₂)₆-O-C(=O)-C(CH₃)=CH₂ 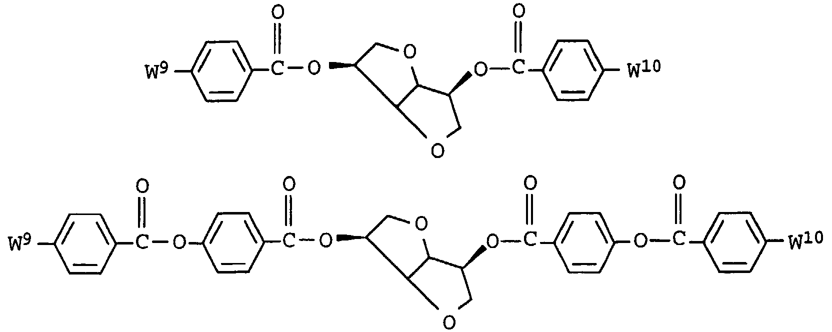
W⁹: CH₂=CH-C(=O)-O-(CH₂)₄-O-C(=O)-O-
W¹⁰: -O-(O=)C-O-(CH₂)₄-O-C(=O)-CH=CH₂ 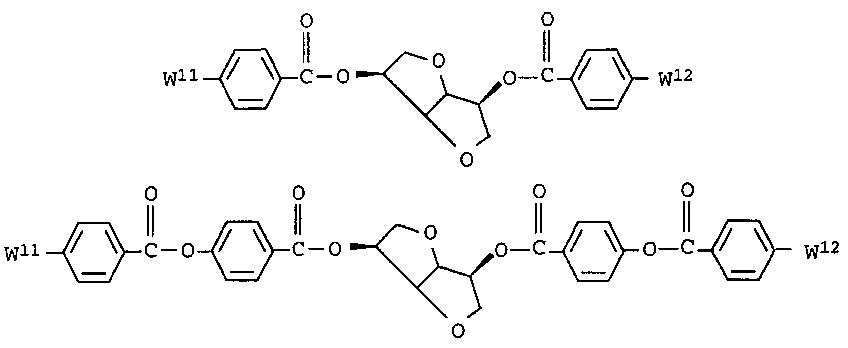
W¹¹: CH₂=CH-C(=O)-O-(CH₂)₆-O-C(=O)-O-
W¹²: -O-(O=)C-O-(CH₂)₆-O-C(=O)-CH=CH₂ 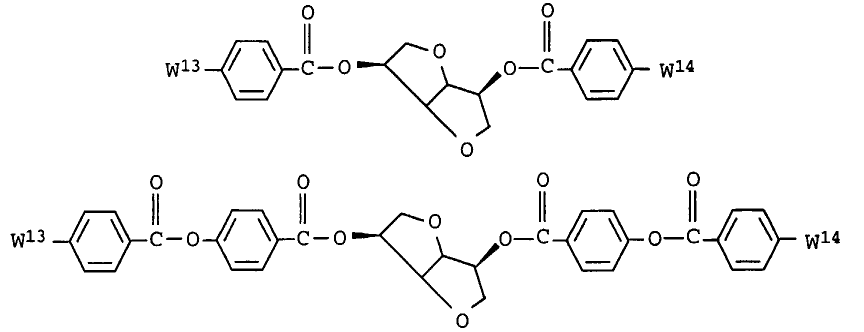
W¹³: CH₂=C(CH₃)-C(=O)-O-(CH₂)₄-O-C(=O)-O-,
W¹⁴: -O-(O=)C-O-(CH₂)₄-O-C(=O)-C(CH₃)=CH₂ 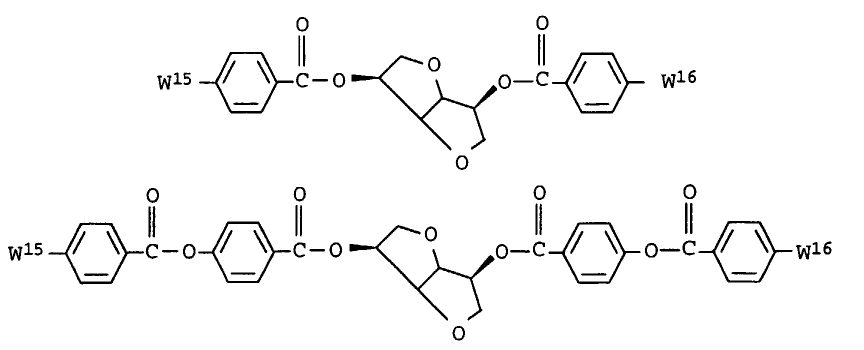
W¹⁵: CH₂=C(CH₃)-C(=O)-O-(CH₂)₆-O-C(=O)-O-,
W¹⁶: -O-(O=)C-O-(CH₂)₆-O-C(=O)-C(CH₃)=CH₂

Die Gewichtsverhältnisse von Komponente II zu Komponente III liegen im Bereich von 99 zu 1 bis 40 zu 60, bevorzugt im Bereich von 99 zu 1 bis 70 zu 30, besonders bevorzugt von 98 zu 2 bis 85 zu 15.

Die Herstellung der oben genannten Verbindungen erfolgt in an sich bekannter Weise gemäß den in DE-A-195 32 408, DE-A-44 08 171, EP-A-0 750 029 sowie in WO 95/16007 beschriebenen Verfahren. Bezüglich näherer Einzelheiten sei auf diese Schriften verwiesen.

Für die erfindungsgemäße Verwendung der oben genannten cholesterisch-flüssigkristdlliflen Zusammensetzungen a)und b) als UV-Filter in kosmetischen und pharmazeutischen Zubereitungen können die in diesen Zusammensetzungen enthaltenen Komponenten der Formel I bis III direkt in die kosmetischen und pharmazeutischen Zubereitungen eingearbeitet werden.

Bevorzugt werden jedoch die erfindungsgemäß verwendeten cholesterisch-flüssigkristallinen Zusammensetzungen in Form von Pigmenten eingesetzt. Diese Pigmente sind dadurch erhältlich, daß die in den cholesterisch-flüssigkristallinen Zusammensetzungen enthaltenen Monomeren I bis III mit Hilfe ihrer polymerisierbaren Gruppen durch radikalische oder ionische Polymerisationsverfahren, welche durch eine photochemische Reaktion gestartet werden können, in hochvernetzte Polymere mit eingefrorener flüssigkristalliner Ordnungsstruktur überführt werden.

Die Herstellung solcher Pigmente ist bekannt und wird u.a. ausführlich in der deutschen Anmeldung P 19738369.6 beschrieben.

Einen Überblick Über Verfahren, orientierte Ausgangsstoffe photochemisch zu vernetzen, findet sich darüberhinaus bei C.G. Roffey, Photopolymerisation of Surface Coatings, (1982) John Willey & Sons, Chichester, S. 137 bis 208.

In einer bevorzugten Ausführungsform werden die dreidimensional vernetzbaren polymerisierbaren Monomeren auf eine Unterlage aufgebracht, auf dieser Unterlage vernetzt und nach dem Vernetzen von der Unterlage abgelöst.

Die zu einem Film vernetzten cholesterisch-flüssigkristallinen Zusammensetzungen lassen sich nach der Polymerisation durch Mahlung auf die jeweils erwünschte Korngröße zerkleinern. Je nach der erwünschten Anwendung bzw. je nach Art der kosmetischen oder pharmazeutischen Formulierung können Korngrößen mit einem Durchmesser von 1 bis 1000 µm hergestellt werden. Bevorzugte Korngrößen liegen im Bereich zwischen 1 und 100 µm, besonders bevorzugt zwischen 15 und 50 µm.

Die Dicke der Pigmente liegt zwischen 1 und 100 µm, bevorzugt zwischen 1 und 50 µm besonders bevorzugt zwischen 1, 5 und 10 µm.

Die cholesterisch-flüssigkristallinen Zusammensetzungen a) bzw. b), die als Ausgangssubstanzen zur Herstellung der Pigmente geeignet sind, besitzen eine verdrillte Struktur mit einer Ganghöhe, die einer Wellenlänge des Lichtes bis zu 450 nm entspricht. Wie in der bevorzugten Ausführungsform gezeigt wird, können sich diese verdrillten Strukturen einer definierten Ganghöhe aus nematischen Strukturen b₁) erhalten werden, indem man ihnen eine chirale Substanz b₂) zusetzt. Art und Anteil der chiralen Substanz bestimmen die Ganghöhe der verdrillten Struktur und damit die Wellenlänge des reflektierten Lichtes. Je nach Chiralität der eingesetzten optisch aktiven Zusatzstoffe kann die Verdrillung der Struktur sowohl links- als auch rechtsgängig sein.

Sogenannte Breitbandreflektoren lassen sich durch einfaches Mischen mehrerer der erfindungsgemäß zu verwendenden cholesterisch flüssigkristallinen Pigmente mit jeweils unterschiedlichen UV-Reflektionsmaxima erzeugen.

Darüber hinaus ist es möglich, durch Mischen von mindestens zwei verschiedenen Pigmenten der cholesterisch-flüssigkristallinen Zusammensetzungen a) und/oder b) mit jeweils entgegengesetzter Verdrillung (Helicität) eine vollständige Reflektion der UV-Strahlen zu erzielen. Pigmente solcher jeweils entgegengesetzt verdrillten, cholesterisch-flüssigkristallinen Strukturen sind beispielsweise durch Zugabe jeweils der einzelnen Spiegelbildisomeren (Enantiomeren) oder Diastereomeren der chiralen Zusatzstoffe b₂) zu dem achiralen flüssigkristallinen polymerisierbaren Monomeren b₁) erhältlich. Die Ganghöhe der jeweils entgegengesetzt verdrillten Strukturen kann dabei gleich oder verschieden sein.

Es ist auch möglich, zunächst die cholesterisch-flüssigkristalline Zusammensetzungen a) oder b) jeweils von entgegengesetzter Verdrillung zu mischen, diese anschließend durch o.g. Vernetzung in die bereits beschriebenen Pigmente zu überführen und als UV-Reflektoren in kosmetischen und pharmazeutischen Formulierungen einzusetzen.

Neben den o.g. Mischungen cholesterisch flüssigkristalliner Pigmente ist es auch möglich Mehrschichtpigmente zu erzeugen, deren einzelne Schichten verschiedene, erfindungsgemäß zu verwendende, dreidimensional vernetzte cholesterisch flüssigkristalline Zusammensetzungen enthalten. Das Design derartiger Mehrschichtpigmente läßt sich vielfältig variieren. So können u.a.
- einzelne Schichten von vernetzten cholesterisch flüssigkristallinen Zusammensetzungen entgegengesetzter Verdrillung oder
- einzelne Schichten von vernetzten cholesterisch flüssigkristallinen Zusammensetzungen gleicher Gangrichtung aber unterschiedlicher Ganghöhe und somit unterschiedlicher Reflektionseigenschaften
übereinander aufgebracht werden.

Bevorzugt sind sogenannte Dreischichtpigmente, bei denen die beiden äußeren Schichten aus jeweils einer der erfindungsgemäß zu verwendenden vernetzten, cholesterisch flüssigkristallinen Zusammensetzungen besteht und die mittlere Schicht beispielsweise eine Bindemittelmatrix enthalten kann, in dem zusätzlich ein weiterer UV-Absorber eingearbeitet sein kann. Einzelheiten bezüglich Herstellung, Eigenschaften und weiterer Bestandteile solcher mehrschichtigen cholesterischen Pigmente sind der deutschen Patentanmeldung P 19738368.8 zu entnehmen.

Gegenstand der Erfindung sind somit auch die oben beschriebenen Pigmente, insbesondere Mehrschichtpigmente, enthaltend die eingangs genannten cholesterisch-flüssigkristallinen Zusammensetzungen.

Ein Vorteil der erfindungsgemäß verwendeten Pigmente liegt darin, daß deren Zusammensetzung so maßgeschneidert eingestellt werden kann, damit mit diesen Pigmenten die gewünschte UV-Reflektion erzielt werden kann ohne aber eine eigene Farbigkeit (im sichtbaren Bereich) zu zeigen.

Ein weiterer Vorteil der Pigmente liegt in ihren physikalischen Eigenschaften. Aufgrund ihrer geringen Dichte (im vergleich beispielsweise zu TiO₂) lassen sich die Pigmente gut in Emulsionen einarbeiten, ohne daß es zu Aggregation oder Separation der Pigmentteilchen kommt.

Die erfindungsgemäß zu verwendenden Pigmente lassen sich durch einfaches Abmischen in die kosmetischen und pharmazeutischen Zubereitungen einarbeiten.

Gegenstand der vorliegenden Erfindung sind weiterhin kosmetische und pharmazeutische Zubereitungen, die 0,1 bis 20 Gew.-%, vorzugsweise 0,5 bis 10 Gew.-%, besonders bevorzugt 1 bis 7 Gew.-%, bezogen auf die gesamte Menge der kosmetischen und pharmazeutischen Zubereitung, eine oder mehrere der cholesterisch-flüssigkristallinen Zusammensetzungen aus a) mindestens einem chiralen flüssigkristallinen, polymerisierbaren Monomeren der allgemeinen Formel I [(Z¹-Y¹-(A¹)ₘ-Y²-M¹-Y³-)ₙ-X] mit dem eine cholesterisch-flüssigkristalline Phase mit einer Ganghöhe von kleiner 450 nm erzielt werden kann oder b) einer Mischung aus mindestens einem achiralen flüssigkristallinen polymerisierbaren Monomeren der allgemeinen Formel II (Z²-Y⁴-(A²)ₒ-Y⁵-M²-Y⁶-(A³)ₚ-Y⁷⁻Z³) und mindestens einem chiralen Zusatzstoff mit der eine cholesterisch-flüssigkristalline Phase mit einer Ganghöhe von kleiner 450 nm erzielt werden kann, zusammen mit an sich für kosmetische und pharmazeutische Zubereitungen bekannten, im UV-A- und UV-B-Bereich absorbierenden Verbindungen als Lichtschutzmittel enthalten. Die Variablen der Formeln I und II sowie die Stoffklasse der eingesetzten chiralen Zusatzstoffe entsprechen dabei sowohl in ihrer allgemeinen als auch in ihrer bevorzugten Ausführungsform den bereits oben geschilderten Erläuterungen.

Bevorzugt sind solche der oben genannten kosmetischen und pharmazeutischen Zubereitungen, die die erfindungsgemäß zu verwendenden cholesterisch-flüssigkristallinen Zusammensetzungen in Form der bereits beschriebenen Pigmente, insbesondere in Form von mehrschichtigen Pigmenten enthalten.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Ethylhexansäurestearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht konnten können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paratfinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Erfindungsgemäß enthalten die erfindungsgemäßen Zubereitungen vorteilhaft ein oder mehrere Antioxidantien. Als günstige, aber dennoch fakultativ zu verwendende Antioxidantien können alle für kosmetische und/oder dermatologische Anwendungen geeigneten oder gebräuchlichen natürlichen, synthetischen und/oder partialsynthetischen Antioxidantien verwendet werden.

Besonders vorteilhaft werden die Antioxidantien gewählt aus der Gruppe, bestehend aus:

Aminosäuren (z.B. Glycin, Histidin, Tyrosin, Trytophan und deren Derivate, Imidazole (z.B. Urocaninsäure) und deren Derivate, Peptide wie D,L,-Carnosin, D-Carnosin, L-Carnosin und deren Derivate (z.B. Anserin), Carotinoide (z.B. β-Carotin, Lycopin) und deren Derivate, Liponsäure und deren Derivate (z.B. Dihydroliponsäure), Aurothioglucose, Propylthiouracil und andere Thioverbindungen (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze sowie Sulfoximinverbindungen (z.B. Buthioninsulfoximin, Homocysteinsulfoximin, Buthioninsulfone, Penta-, Hexa-, Heptathioninsulfoxmin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA, EGTA und deren Derivate, ungesättigte Fettsäuren und deren Derivate (z.B. γ-Linolensäure, Linolsäure, Ölsäure), Folsäure und deren Derivate (z.B. 5-Methyltetrahydro-folsäure), Ubichinon und Ubichinol und deren Derivate, Vitamin C und deren Derivate (z.B. Ascorbylpalmitat, Ascorbylphosphate, Ascorbylacetate), Tocopherole und Derivate (z.B. Tocopherylacetat, Tocotrienol), Vitamin A und Derivate (z.B. Vitamin A-Palmitat), Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Stilbene und deren Derivate.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Zubereitung - betragen. Die Herstellung der Zubereitung kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden cholesterisch-flüssigkristallinen Zusammensetzungen 10 bis 90 Gew.-%, bevorzugt 20 bis 70 Gew.-% bezogen auf die Gesamtmenge von UV-B und UV-A absorbierenden Substanzen.

Als UV-Filtersubstanzen, die in Kombination mit den erfindungsgemäß zu verwendenden cholesterisch-flüissigkristallinen Zusammensetzungen angewandt werden, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexyl-ester | 6197-30-4 |
| 21 | Menthyl-o-aminobenzoate oder: | 134-09-8 |
| | 5-Methyl-2-(1-methylethyl)-2-aminobenzoate | |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxy-benzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: | 4732-70-1 |
| | 3,4-dimethoxy-phenyl-glyoxal-saures Natrium | |
| 27 | 3-(4'Sulfo)benzyliden-bornan-2-on und seine Salze | 56039-58-8 |
| 28 | 4-tert.-Butyl-4 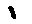 -methoxy-dibenzoylmethan | 70356-09-1 |
| 29 | 2,2 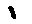 ,4,4 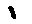 -Tetrahydroxybenzophenon | 131-55-5 |

Schließlich sind auch mikronisierte Pigmente wie Titandioxid und Zinkoxid zu nennen.

Zum Schutz menschlicher Haare vor UV-Strahlen können die erfindugsgemäß verwendeten cholesterisch flüssigkristallinen Zusammensetzungen a) und/oder b) in Shampoos, Lotionen, Gelen, Haarsprays, Aerosol-Schaumcremes oder Emulsionen in Konzentrationen von 0,1 bis 20 Gew.-%, bevorzugt 0,5 bis 10 Gew.-%, besonders bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Zusammensetzungen zeichnen sich in der Regel durch ein besonders hohes Reflektionsvermögen im Bereich der UV-A- und UV-B-Strahlung mit scharfer Bandenstruktur aus. Weiterhin lassen sie sich leicht in kosmetische und pharmazeutischen Formulierungen einarbeiten. Außerdem zeichnen sie sich besonders durch ihre hohe Photostabilität, und die damit hergestellten Zubereitungen durch ihr angenehmes Hautgefühl aus.

Die UV-Filterwirkung der erfindungsgemäßen verwendeten cholesterisch flüissigkristallinen Zusammensetzungen a) und/oder b) kann auch zur Stabilisierung von Wirk- und Hilfsstoffen in kosmetischen und pharmazeutischen Formulierungen ausgenutzt werden.

Die folgenden Beispiele sollen die erfinderische Verwendung der cholesterisch flüssigkristallinen Zusammensetzungen näher erläutern.

### Beispiel 1

### Herstellung cholesterischer Pigmente (Pigment 1)

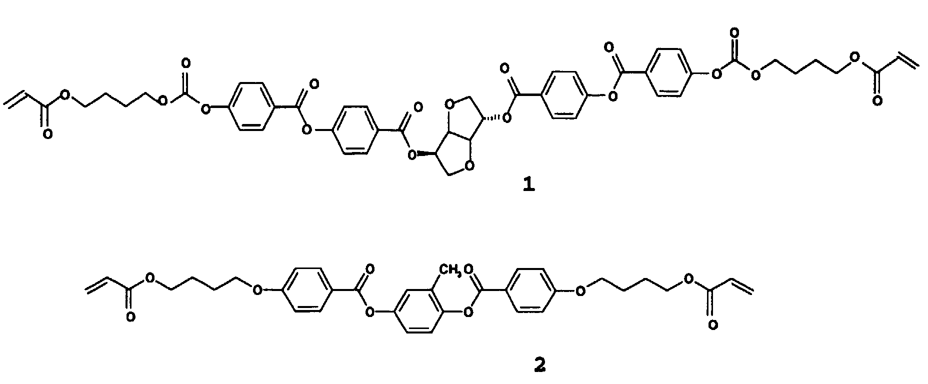

Es wurde eine cholesterisch flüssigkristalline Mischung eingesetzt, die als chirales Monomer eine Verbindung der oben angegebenen Formel 1 und als achirales, nematisches Monomer eine Verbindung der oben angegebenen Formel 2 enthielt. Das unverdünnte cholesterische Gemisch enthielt 94,8 Gew.-% der achiralen, nematischen Verbindung, 5,2 Gew.-% der chiralen Verbindung und als Photoinitiator 2 Gew.-%, bezogen auf die cholesterisch flüssigkristalline Mischung, 1-Hydrocyclohexylphenylketon, das unter der Bezeichnung Irgacure 184 vertrieben wird. Die Mischung zeigte ein λₘₐₓ = 350 nm (T = 70°C).

Zur Herstellung der Pigmente wurde diese Mischung in Methylethylketon gelöst und zur Beschichtung auf eine Polyethylenterephthalfolie aufgetragen. Die Beschichtung erfolgte nach einem in DE-A 19 63 8797 beschriebenen Verfahren.

Die Dicke der cholesterischen Schicht betrug 2,5 µm. Nach Abdampfen des Lösungsmittels bei 70°C wurde die Schicht durch UV-Bestrahlung vernetzt und ausgehärtet. Die so erhaltene gehärtete Cholesterenschicht wurde vom Träger abgelöst und durch Vermahlen und anschließendes Sieben klassiert. Die Korngröße der Pigmentpartikel lag im Bereich < 50 µm.

### Beispiel 2

### Herstellung cholesterischer Pigmente (Pigment 2)

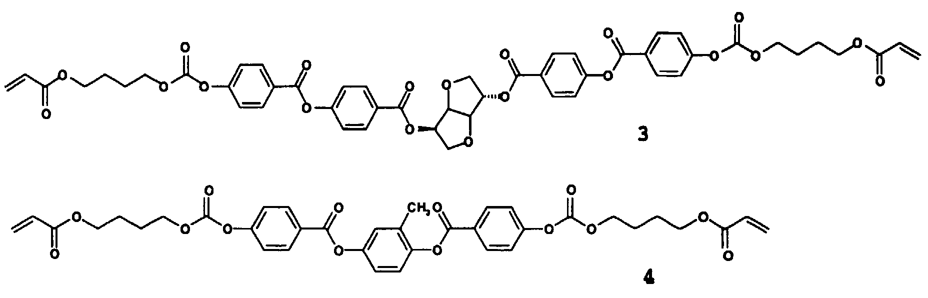

Analog Beispiel 1 wurden Pigmente hergestellt, die als chirales Monomer eine Verbindung der Formel 3 (5,2 Gew.-%) und als achirales, nematisches Monomer eine Verbindung der Formel 4 (94,8 Gew.-%) enthielten. Als Photoinitiator wurden 2 Gew.-% Irgacure 184, bezogen auf die cholesterisch flüssigkristalline Mischung, eingesetzt. Die Mischung zeigte ein λₘₐₓ = 350 nm (T = 23°C).

### Beispiel 3

### Herstellung cholesterischer Pigmente (Pigment 3)

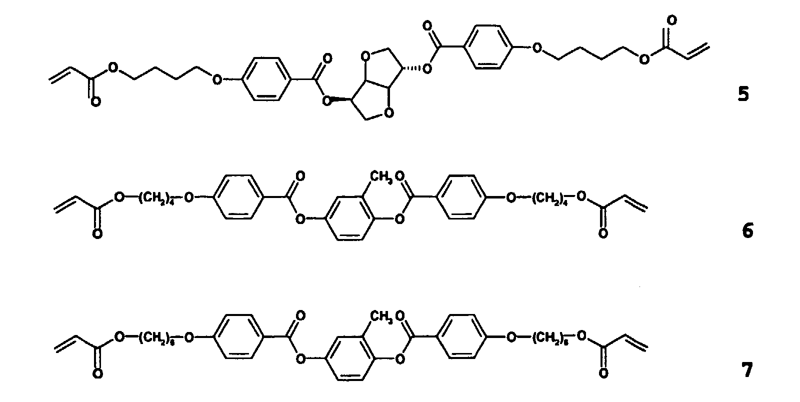

Analog Beispiel 1 wurden Pigmente hergestellt, die als chirales Monomer eine Verbindung der Formel 5 (11 Gew.-%) und als achirales, nernatisches Monomer eine 1:1 Mischung der Verbindungen der Formeln 6 und 7 (89 Gew.-%) enthielten. Die Menge an Irgacure 184 betrug 2 Gew.-%, bezogen auf die cholesterisch flüssigkristalline Mischung. Die Mischung zeigte ein λₘₐₓ = 355 nm (T = 23°C).

### Zubereitungen

### Beispiel 4

### Zusammensetzung für die Lippenpflege

### Massengehalt

### (Gew.-%)

- ad 100: Eucerinum anhydricum
- 10,00: Glycerin
- 5,00: Pigment 1
- 8,00: Octyl Methoxycinnamat
- 5,00: Zink Oxid
- 4,00: Castoröl
- 4,00: Pentaerythrithyl Stearat/Caprat/Caprylat Adipat
- 3,00: Glyceryl Stearat SE
- 2,00: Bienenwachs
- 0,50: Tocopheryl Acetat
- 2,00: Microkristallines Wachs
- 2,00: Quaternium-18 Bentonit
- 1,50: PEG-45/Dodecyl Glycol Copolymer

### Beispiel 5

### Zusammensetzung für die Lippenpflege

### Massengehalt

### (Gew.-%)

- ad 100: Eucerinum anhydricum
- 10,00: Glycerin
- 5,00: Pigment 3
- 8,00: Octyl Methoxycinnamat
- 10,00: Zink Oxid
- 4,00: Castoröl
- 4,00: Pentaerythrithyl Stearat/Caprat/Caprylat Adipat
- 3,00: Glyceryl Stearat SE
- 2,00: Bienenwachs
- 0,50: Tocopheryl Acetat
- 2,00: Microkristallines Wachs
- 2,00: Quaterniurn-18 Bentonit
- 1,50: PEG-45/Dodecyl Glycol Copolymer

### Beispiel 6

### Zusammensetzung für Sunblocker mit Mikropigmenten

### Massengehalt

### (Gew.-%)

- ad 100: Wasser
- 10,00: Octyl Methoxcinnamat
- 6,00: PEC-7-Hydrogenated Castor Öl
- 6,00: Titanium Dioxid
- 5,00: Pigment 1
- 5,00: Mineral Öl
- 5,00: Isoaxnyl p-Methoxycinnamat
- 5,00: Propylen Glycol
- 3,00: Jojoba Öl
- 3,00: 4-Methylbenzyliden Campher
- 2,00: PEG-45/Dodecyl Glycol Copolymer
- 1,00: Dimethicon
- 0,50: PEO-40-Hydrogenated Castor Öl
- 0,50: Tocopheryl Acetat
- 0,50: Phenoxyethanol
- 0,20: EDTA

### Beispiel 7

### Zusammensetzung für Simnblocker mit Mikropigmenten

### Massengehalt

### (Gew.-%)

- ad 100: Wasser
- 10,00: Octyl Methoxcinnamat
- 6,00: PEG-7-Hydrogenated Castor Öl
- 6,00: Titanium Dioxid
- 5,00: Pigment 3
- 5,00: Mineral Öl
- 5,00: Isoamyl p-Methoxycinnamat
- 5,00: Propylen Glycol
- 3,00: Jojoba Öl
- 3,00: 4-Methylbenzyliden Campher
- 2,00: PEG-45/Dodecyl Glycol Copolymer
- 1,00: Dimethicon
- 0,50: PEG-40-Hydrogenated Castor Öl
- 0,50: Tocopheryl Acetat
- 0,50: Phenoxyethanol
- 0,20: EDTA

### Beispiel 8

### Fettfreies Gel

### Massengehalt

### (Gew.-%)

- ad 100: Wasser
- 8,00: Octyl Methoxycinnamat
- 5,00: Pigment 1
- 5,00: Glycerin
- 5,00: PEG-25 PABA
- 1,00: 4-Methylbenzyliden Campher
- 0,40: Acrylate C10-C30 Alkyl Acrylat Crosspolymer
- 0,30: Imidazolidinyl Urea
- 0,25: Hydroxyethyl Cellulose
- 0,25: Sodium Methylparaben
- 0,20: Disodium EDTA
- 0,15: Fragrance
- 0,15: Sodium Propylparaben
- 0,10: Sodium Hydroxid

### Beispiel 9

### Fettfreies Gel

### Massengehalt

### (Gew.-%)

- ad 100: Wasser
- 8,00: Octyl Methoxycinnamat
- 7,00: Pigment 3
- 5,00: Glycerin
- 5,00: PEG-25 PABA
- 1,00: 4-Methylbenzyliden Campher
- 0,40: Acrylate C10-C30 Alkyl Acrylat Crosspolymer
- 0,30: Imidazolidinyl Urea
- 0,25: Hydroxyethyl Cellulose
- 0,25: Sodium Methylparaben
- 0,20: Disodium EDTA
- 0,15: Fragrance
- 0,15: Sodium Propylparaben
- 0,10: Sodium Hydroxid

### Beispiel 10

### Sonnencreme (LSF 20)

### Massengehalt

### (Gew.-%)

- ad 100: Wasser
- 8,00: Octyl Methoxycinnamat
- 6,00: PEG-7-Hydrogenated Castor Öl
- 5,00: Pigment 1
- 6,00: Mineral Öl
- 5,00: Zink Oxid
- 5,00: Isopropyl Palmitat
- 5,00: Imidazolidinyl Urea
- 3,00: Jojoba Öl
- 2,00: PEG-45/Dodecyl Glycol Copolymer
- 1,00: 4-Methylbenzyliden Campher
- 0,60: Magnesium Stearat
- 0,50: Tocopheryl Acetat
- 0,25: Methylparaben
- 0,20: Disodium EDTA
- 0,15: Propylparaben

### Beispiel 11

### Sonnencreme (LSF 20)

### Massengehalt

### (Gew.-%)

- ad 100: Wasser
- 8,00: Octyl Methoxycinnamat
- 6,00: PEG-7-Hydrogenated Castor Öl
- 5,00: Pigment 3
- 6,00: Mineral Öl
- 5,00: Zink Oxid
- 5,00: Isopropyl Palmitat
- 5,00: Imidazolidinyl Urea
- 3,00: Jojoba Öl
- 2,00: PEG-45/Dodecyl Glycol Copolymer
- 1,00: 4-Methylbenzyliden Campher
- 0,60: Magnesium Stearat
- 0,50: Tocopheryl Acetat
- 0,25: Methylparaben
- 0,20: Disodium EDTA
- 0,15: Propylparaben

### Beispiel 12

### Sonnencreme wasserfest

### Massengehalt

### (Gew.-%)

- ad 100: Wasser
- 8,00: Octyl Methoxycinnamat
- 5,00: PEG-7-Hydrogenated Castor Öl
- 5,00: Propylene Glycol
- 4,00: Isopropyl Palmitat
- 4,00: Caprylic/Capric Triglycerid
- 5,00: Pigment 1
- 4,00: Glycerin
- 3,00: Jojoba Öl
- 2,00: 4-Methylbenzyliden Campher
- 1,50: PEG-45/Dodecyl Glycol Copolymer
- 1,50: Dimethicon
- 0,70: Magnesium Sulfat
- 0,50: Magnesium Stearat
- 0,50: Tocopheryl Acetat
- 0,15: Fragrance

### Beispiel 13

### Sonnencreme wasserfest

### Massengehalt

### (Gew.-%)

- ad 100: Wasser
- 8,00: Octyl Methoxycinnamat
- 5,00: PEG-7-Hydrogenated Castor Öl
- 5,00: Propylene Glycol
- 4,00: Isopropyl Palmitat
- 4,00: Caprylic/Capric Triglycerid
- 5,00: Pigment 3
- 4,00: Glycerin
- 3,00: Jojoba Öl
- 2,00: 4-Methylbenzyliden Campher
- 1,50: PEG-45/Dodecyl Glycol Copolymer
- 1,50: Dimethicon
- 0,70: Magnesium Sulfat
- 0,50: Magnesium Stearat
- 0,50: Tocopheryl Acetat
- 0,15: Fragrance

### Beispiel 14

### Sonnenmilch (LSF 6)

### Massengehalt

### (Gew.-%)

- ad 100: Wasser
- 10,00: Mineral Öl
- 6,00: PEG-7-Hydrogenated Castor Öl
- 5,00: Isopropyl Palmitat
- 3,50: Octyl Methoxycinnamat
- 3,00: Pigment 1
- 3,00: Caprylic/Capric Triglycerid
- 3,00: Jojoba Öl
- 2,00: PEG-45/Dodecyl Glycol Copolymer
- 0,70: Magnesium Sulfat
- 0,60: Magnesium Stearat
- 0,50: Tocopheryl Acetat
- 0,30: Glycerin
- 0,25: Methylparaben
- 0,15: Propylparaben

### Beispiel 15

### Sonnenmilch (LSF 6)

### Massengehalt

### (Gew.-%)

- ad 100: Wasser
- 10,00: Mineral Öl
- 6,00: PEG-7-Hydrogenated Castor Öl
- 5,00: Isopropyl Palmitat
- 3,50: Octyl Methoxycinnamat
- 3,00: Pigment 3
- 3,00: Caprylic/Capric Triglycerid
- 3,00: Jojoba Öl
- 2,00: PEG-45/Dodecyl Glycol Copolymer
- 0,70: Magnesium Sulfat
- 0,60: Magnesium Stearat
- 0,50: Tocopheryl Acetat
- 0,30: Glycerin
- 0,25: Methylparaben
- 0,15: Propylparaben

## Patentansprüche

1. Verwendung von cholesterisch-flüssigkristallinen Zusammensetzungen, enthaltend
a) mindestens ein chirales flüssigkristallines, polymerisierbares Monomeres der allgemeinen Formel I,
[Z¹-Y¹-(A¹)ₘ-Y²-M¹-Y³-]ₙ-X I
mit dem eine cholesterisch-flüssigkristalline Phase mit einer Ganghöhe von kleiner 450 nm erzielt werden kann, in der die variablen unabhängig voneinander folgende Bedeutung haben:
A¹ ein Spacer einer Kettenlänge von 1 bis 30 C-Atomen,
Y¹ bis Y³ eine chemische Bindung, -O-, -S-, -C(=O)-O-, -O-C(=O)-, -CH=CH-C(=O)-O-, -O-C(=O)-O-, -C(=O)-N(R)- oder -(R)N-C(=O)-, -CH₂-O-, -O-CH₂-, -CH=N-, -N=CH- oder -N=N-,
M¹ eine mesogene Gruppe,
R Wasserstoff, C₁-C₄-Alkyl,
Z¹ Wasserstoff, C₁-C₄-Alkyl, eine polymerisierbare Gruppe oder ein Rest, der eine polymerisierbare Gruppe trägt,
X ein n-wertiger chiraler Rest,
m 0 oder 1,
n 1 bis 6 wobei die Reste Z¹, Y¹, Y², Y³, A¹ und M¹ gleich oder verschieden sein können und mindestens ein Rest Z¹ eine polymerisierbare Gruppe oder einen Rest, der eine polymerisierbare Gruppe enthält, darstellt, wenn n größer als 1 ist,
oder
b) eine Mischung aus
b₁) mindestens einem achiralen flüissigkristallinen polymerisierbaren Monomeren der allgemeinen Formel II
Z²-Y⁴-(A²)ₒ-Y⁵-M²-Y⁶-(A³)ₚ-Y⁷-Z³ II
in der die variablen unabhängig voneinander folgende Bedeutung haben:
A² und A³ ein Spacer einer Kettenlänge von 1 bis 30 C-Atomen,
M² eine mesogene Gruppe,
Y⁴ bis Y⁷ eine chemische Bindung, -O-, -S-, -C(=O)-O-, -O-C(=O)-, -CH=CH-C(=O)-O-, -O-C(=O)-O-, -C(=O)-N(R¹)- oder -(R¹)N-C(=O)-, -CH₂-O-, -O-CH₂-, -CH=N-, -N=CH- oder -N=N-,
R¹ Wasserstoff, C₁-C₄-Alkyl,
o,p 0 oder 1
Z² und Z³ Wasserstoff, C₁-C₄-Alkyl, eine polymerisierbare Gruppe oder ein Rest, der eine polymerisierbare Gruppe trägt,
wobei mindestens eine der variablen Z² oder Z³ eine polymerisierbare Gruppe oder einen Rest, der eine polymerisierbare Gruppe trägt, darstellt und
b₂₎ mindestens einem chiralen Zusatzstoff, mit dem eine cholesterisch-flüssigkristalline Phase mit einer Ganghöhe von kleiner 450 nm erzielt werden kann,
als UV-Filter in kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

2. verwendung von choiesterisch-flüssigkristallinen Zusammensetzungen nach Anspruch 1, enthaltend als chiralen Zusatzstoff b₂) mindestens ein chirales polymerisierbares Monomer der allgemeinen Formel III,
[Z¹-Y²-(A¹)ₘ-Y²-M³-Y³-]ₙ-X III
in der die Variablen Z¹, Y¹, Y², Y³, A¹, X, m und n die in Anspruch 1 angegebene Bedeutung haben und M³ ein zweiwertiger Rest ist, der mindestens ein hetero- oder isocyclisches Ringsystem enthält.

3. Verwendung von cholesterisch-flüssigkristallinen Zusammensetzungen nach den Ansprüchen 1 und 2 mit
A¹ bis A³ ein Spacer einer Kettenlänge von 1 bis 6 C-Atomen;
M¹ bis M3 ein Rest aus der Gruppe, bestehend aus: 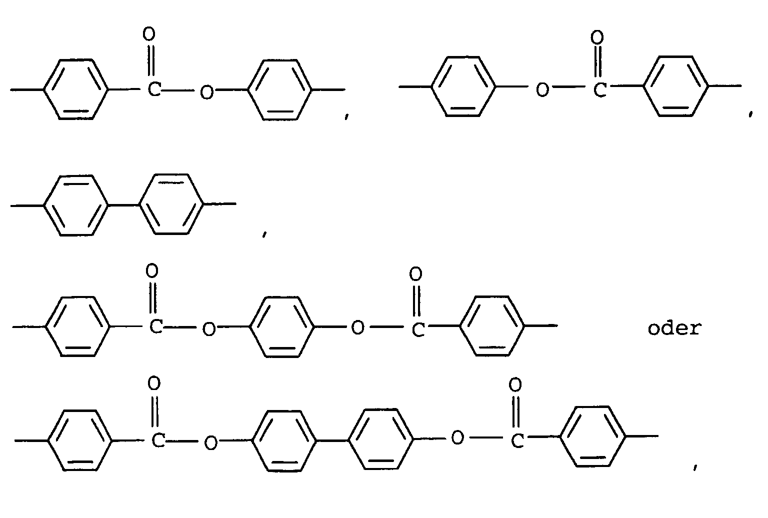 wobei jeder aromatische Ring bis zu drei gleiche oder verschiedene Substituenten aus der folgenden Gruppe tragen kann: Wasserstoff, C₁-C₂₀-Alkyl, C₁-C₂₀-Alkoxy, C₁-C₂₀-Alkoxycarbonyl, N-C₁-C₂₀-Alkylaminocarbonyl, C₁-C₂₀-Alkylcarbonyl, C₁-C₂₀-Alkylcarbonyloxy, N-C₁-C₂₀-Alkylcarbonylamino, Formyl, Halogen, Cyan, Hydroxy und Nitro;
X einen chiralen Rest aus der Gruppe, bestehend aus: 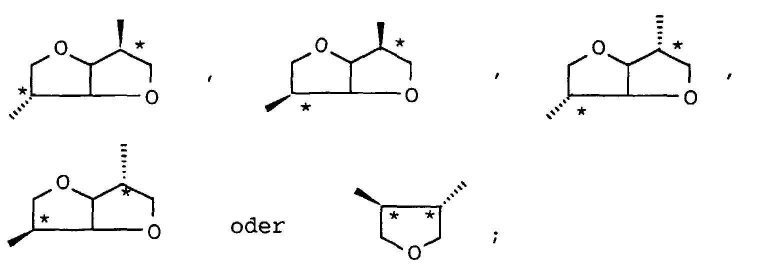
n 2
o,p 1.

4. Verwendung von cholesterisch-tlüssigkristallinen Zusammensetzungen nach den Ansprüchen 1 bis 3 als photostabile UV-Reflektoren.

5. Verwendung von cholesterisch-flüssigkristallinen Zusammensetzungen definiert gemäß Anspruch 1 als UV-Stabilisatoren in kosmetischen und pharmazeutischen Formulierungen.

6. Verwendung von cholesterisch-flüssigkristallinen Zusammensetzungen nach den Ansprüchen 1 bis 5 in Form von Pigmenten.

7. Pigmente enthaltend polymerisierte cholesterisch-flüssigkristalline Zusammensetzungen gemäß Anspruch 1.

8. Pigmente nach Anspruch 7, dadurch gekennzeichnet, daß es sich um Mehrschichtpigmente handelt.

9. Lichtschutzmittel enthaltende kosmetische und pharmazeutische Zubereitungen zum Schutz der menschlichen Epidermis oder menschlichen Haare gegen UV-Licht im Bereich von 280 bis 400 nm, dadurch gekennzeichnet, daß sie in einem kosmetisch und pharmazeutisch geeigneten Träger, allein oder zusammen mit an sich für kosmetische und pharmazeutische Zubereitungen bekannten im UV-Bereich absorbierenden Verbindungen, als photostabile UV-Filter wirksame Mengen von cholesterischflüssigkristallinen Zusammensetzungen gemäß Anspruch 1 enthalten.

10. Kosmetische und pharmazeutische Zubereitungen nach Anspruch 9, enthaltend als UV-Filter cholesterisch-flüssigkristalline Zusammensetzungen gemäß Anspruch 3.

11. Kosmetische und pharmazeutische Zubereitungen nach Anspruch 9, enthaltend als UV-Filter cholesterisch-flüssigkristalline Zusammensetzungen in Form von Pigmenten.

12. Kosmetische und pharmazeutische Zubereitungen nach Anspruch 9, enthaltend als UV-Filter cholesterisch-flüssigkristalline Zusammensetzungen in Form von mehrschichtigen Pigmenten.
